(19) Europäisches Patentamt / European Patent Office / Office européen des brevets

(11) **EP 4 174 167 A1**

(12) **EUROPEAN PATENT APPLICATION**
published in accordance with Art. 153(4) EPC

(43) Date of publication:
**03.05.2023 Bulletin 2023/18**

(21) Application number: **21831803.8**

(22) Date of filing: **23.06.2021**

(51) International Patent Classification (IPC):
**C12N 1/20** (2006.01)    **A01P 3/00** (2006.01)
**A01N 63/20** (2020.01)

(52) Cooperative Patent Classification (CPC):
**A01N 63/20; A01P 3/00; C12N 1/20**

(86) International application number:
**PCT/JP2021/023736**

(87) International publication number:
**WO 2022/004515 (06.01.2022 Gazette 2022/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.06.2020   JP 2020111696**

(83) **Declaration under Rule 32(1) EPC (expert solution)**

(71) Applicant: **SUMITOMO CHEMICAL COMPANY, LIMITED**
**Tokyo 103-6020 (JP)**

(72) Inventors:
• **AOKI, Mikio**
  **Osaka-shi, Osaka 554-8558 (JP)**
• **OZAWA, Naoya**
  **Osaka-shi, Osaka 554-8558 (JP)**

(74) Representative: **Vossius & Partner Patentanwälte Rechtsanwälte mbB**
**Siebertstraße 3**
**81675 München (DE)**

Remarks:
The complete document including Reference Table(s) and the Sequence Listing(s) can be downloaded from the EPO website

(54) **NOVEL MICROORGANISM BELONGING TO GENUS LACTOBACILLUS, AND AGENT AND METHOD FOR CONTROLLING PLANT DISEASE CAUSED BY RALSTONIA SOLANACEARUM OR RALSTONIA PSEUDOSOLANACEARUM**

(57)    Provided are a bacterium having a 16S rRNA gene that contains a nucleotide sequence with 98.80 % or more identity to the nucleotide sequence of SEQ ID No: 1 and having the ability to assimilate mannitol and the ability to control a plant disease and a bacterium with Accession Number of NITE BP-03197, NITE BP-03198, NITE BP-03199, NITE BP-03200, NITE BP-03201, or NITE BP-03202, and a control agent against a plant disease caused by *Ralstonia solanacearum* or *Ralstonia pseudosolanacearum,* containing bacterial cells or a cell culture product of the bacterium, or an extract thereof.

FIG.8

**Description**

TECHNICAL FIELD

The present invention relates to new microorganisms belonging to the genus *Lactobacillus,* and a control agent and a control method against a plant disease caused by *Ralstonia solanacearum* or *Ralstonia pseudosolanacearum.*

BACKGROUND ART

*Ralstonia solanacearum* and *Ralstonia pseudosolanacearum* infect 200 or more species of plants to cause bacterial wilt in a *Solanaceae* plant such as tomato, eggplant, green pepper, and potato, a *Cucurbitaceae* plant such as cucumber, and an ornamental plant such as statice and Texas bluebell, or the like, and damping-off in tobacco. Such a plant disease caused by *Ralstonia solanacearum* and *Ralstoniapseudosolanacearum* occurs on a global scale and has a great economical loss.

As a method for controlling a plant disease, Japanese Patent Laying-Open No. 2009-201459 A (Patent Literature 1) discloses a method for controlling soft rot caused by Erwinia carotovora by a composition containing *Lactobacillus kyotoensis* FERM P-21500 or further containing *Lactobacillus plantarum* FERM P-21501 in addition to it, for example. J Gen Plant Pathol 70:115-119 (2004) (Non Patent Literature 1) discloses that infection of tomato with *Ralstonia solanacearum* is inhibited by grafting with resistant tomato. Japanese Patent Laying-Open No. 2012-211124 A (Patent Literature 2) discloses a method for controlling bacterial wilt by L-amino acid absorption into a target plant such as tomato.

CITATION LIST

PATENT LITERATURE

[0004]

　　PTL 1: Japanese Patent Laying-Open No. 2009-201459 A
　　PTL 2: Japanese Patent Laying-Open No. 2012-211124 A

NON PATENT LITERATURE

NPL 1: J Gen Plant Pathol 70:115-119 (2004)

SUMMARY OF INVENTION

TECHNICAL PROBLEM

Up to now, an attempt to control a plant disease caused by *Ralstonia solanacearum* and *Ralstoniapseudosolanacearum* using a chemical fumigant, a bactericide, a resistant variety, or a resistance inducer has been made. However, the use of such a chemical fumigation method is limited due to its limited effective range as well as its toxicity to a human and its large environmental burden. Validamycin can be applied as a bactericide against bacterial wilt of potato, but no bactericide that can be used for bacterial wilt of another plant has been reported. In the case of using a resistant variety, there has been a problem of poor taste and poor productivity. In the case of using a rootstock, there has been a problem of poor control effect. In the case of using a resistance inducer, there has been a problem where it has no direct antibacterial activity against disease bacteria and its effect varies depending on the crop variety.

It is an object of the present invention to provide a new control agent and a new control method against a plant disease caused by *Ralstonia solanacearum* or *Ralstonia pseudosolanacearum.*

SOLUTION TO PROBLEM

The present invention relates to [1] to [14] below, for example.

　　[1] A bacterium having a 16S rRNA gene that contains a nucleotide sequence with 98.80 % or more identity to the nucleotide sequence of SEQ ID No: 1 and having the ability to assimilate mannitol and the ability to control a plant disease.
　　[2] A bacterium comprising a 16S rRNA gene that contains a nucleotide sequence with 99.90 % or more identity to the nucleotide sequence of SEQ ID No: 1 and having the ability to control a plant disease.

[3] The bacterium according to [1] or [2], having a 16S rRNA gene that contains the nucleotide sequence of SEQ ID No: 1.

[4] A bacterium with Accession Number of NITE BP-03197, NITE BP-03198, NITE BP-03199, NITE BP-03200, NITE BP-03201, or NITE BP-03202.

[5] Bacterial cells or a cell culture product of the bacterium according to any one of [1] to [4], or an extract thereof.

[6] A control agent against a plant disease caused by *Ralstonia solanacearum* or *Ralstonia pseudosolanacearum*, comprising bacterial cells or a cell culture product of the bacterium according to any one of [1] to [4], or an extract thereof.

[7] A method for controlling a plant disease caused by *Ralstonia solanacearum* or *Ralstonia pseudosolanacearum*, comprising applying the control agent according to [6].

[8] A disinfectant for a plant, a nutrient solution, soil, a nutriculture material, or a soil culture material contaminated by *Ralstonia solanacearum* or *Ralstonia pseudosolanacearum*, comprising bacterial cells or a cell culture product of the bacterium according to any one of [1] to [4], or an extract thereof.

[9] A method for disinfecting a plant, a nutrient solution, soil, a nutriculture material, or a soil culture material contaminated by *Ralstonia solanacearum* or *Ralstonia pseudosolanacearum*, comprising using the disinfectant according to [8].

[10] A growth inhibitor for *Ralstonia solanacearum* or *Ralstonia pseudosolanacearum*, comprising bacterial cells or a cell culture product of the bacterium according to any one of [1] to [4], or an extract thereof.

[11] A method for inhibiting growth of *Ralstonia solanacearum* or *Ralstonia pseudosolanacearum*, comprising using the growth inhibitor according to [10].

[12] A method for cultivating a plant, comprising cultivating a plant in a nutrient solution or soil that contains bacterial cells or a cell culture product of the bacterium according to any one of [1] to [4], or an extract thereof.

[13] A control agent against a plant disease caused by *Ralstonia solanacearum* or *Ralstonia pseudosolanacearum*, comprising bacterial cells or a cell culture product of bacterium belonging to *Lactobacillus plantarum*, or an extract thereof.

[14] A method for controlling a plant disease caused by *Ralstonia solanacearum* or *Ralstonia pseudosolanacearum*, comprising applying the control agent according to [13].

## ADVANTAGEOUS EFFECTS OF INVENTION

The present invention enables a plant disease caused by *Ralstonia solanacearum* or *Ralstoniapseudosolanacearum* to be controlled.

## BRIEF DESCRIPTION OF DRAWINGS

FIG. 1 includes views showing (A) the colony shape and (B) the result of gram staining of NITE BP-03197 in Experiment 2.

FIG. 2 includes views showing (A) the colony shape and (B) the result of gram staining of NITE BP-03198 in Experiment 3.

FIG. 3 includes views showing (A) the colony shape and (B) the result of gram staining of NITE BP-03199 in Experiment 4.

FIG. 4 includes views showing (A) the colony shape and (B) the result of gram staining of NITE BP-03200 in Experiment 5.

FIG. 5 includes views showing (A) the colony shape and (B) the result of gram staining of NITE BP-03201 in Experiment 6.

FIG. 6 includes views showing (A) the colony shape and (B) the result of gram staining of NITE BP-03202 in Experiment 7.

FIG. 7 is a diagram illustrating a test for evaluating the antibacterial activity in Experiment 8.

FIG. 8 shows the result of a test for evaluating the antibacterial activity of NITE BP-03197, NITE BP-03198, NITE BP-03199, NITE BP-03200, NITE BP-03201, or NITE BP-03202 in Experiment 8.

FIG. 9 is a diagram illustrating a test for evaluating the antibacterial activity in Experiment 9.

FIG. 10 shows the result of a test for evaluating the antibacterial activity of NITE BP-03197, NITE BP-03198, NITE BP-03199, NITE BP-03200, NITE BP-03201, or NITE BP-03202 in Experiment 9.

FIG. 11 shows the result of a test for evaluating the antibacterial activity of NITE BP-03197, NITE BP-03198, NITE BP-03199, NITE BP-03200, NITE BP-03201, or NITE BP-03202 in Experiment 10.

FIG. 12 is a diagram illustrating a test for evaluating the antibacterial activity in Experiment 11.

FIG. 13 shows the result of a test for evaluating the antibacterial activity of NITE BP-03197, NITE BP-03198, NITE BP-03199, NITE BP-03200, NITE BP-03201, or NITE BP-03202 in Experiment 11.

FIG. 14 is a graph showing the effect of controlling bacterial wilt by the cell culture supernatant of NITE BP-03201 in Experiment 13.

FIG. 15 is a graph showing the effect of controlling bacterial wilt by the cell culture supernatant of NITE BP-03199 or NITE BP-03200 in Experiment 13.

## DESCRIPTION OF EMBODIMENTS

Hereinafter, the embodiments of the present invention will be described in detail. The present invention is not limited to the following embodiments.

[Microorganism belonging to genus *Lactobacillus*]

Bacteria a according to one embodiment of the present invention comprise a 16S rRNA gene that contains a nucleotide sequence with an identity of 98.80 % or more to the nucleotide sequence of SEQ ID No: 1 and have the ability to assimilate mannitol and the ability to control a plant disease.

Bacteria a preferably comprise a 16S rRNA gene that contains a nucleotide sequence with an identity of 99.00 % or more, 99.20 % or more, 99.50 % or more, 99.80 % or more, or 99.90 % or more, to the nucleotide sequence of SEQ ID No: 1. Bacteria a may include a 16S rRNA gene that contains the nucleotide sequence of SEQ ID No: 1. Examples of the bacteria comprising a 16S rRNA gene that contains a nucleotide sequence with an identity of 98.80 % or more to the nucleotide sequence of SEQ ID No: 1 include bacteria belonging to the genus *Lactobacillus* (lactic acid bacteria).

Bacteria a may include a 16S rRNA gene that contains a nucleotide sequence with one or several bases replaced, deleted, or added in the nucleotide sequence of SEQ ID No: 1. The phrase, one or several bases, for example, can mean 1 to 18 bases, preferably 1 to 10 bases, more preferably 1 to 5 bases.

Bacteria a may include a 16S rRNA gene that contains a continuous sequence of about 15 bases or more, preferably about 18 to about 500 bases, more preferably about 18 to about 200 bases, further preferably about 18 to about 50 bases contained in the nucleotide sequence of SEQ ID No: 1 or a nucleotide sequence that hybridizes to a complementary sequence thereof under stringent conditions. The stringent conditions refer to conditions in which a non-specific hybrid is not formed. Examples thereof include conditions in which washing is performed one or more times in 60°C, 1 × SSC, and 0.1% SDS, preferably 68°C, 0.1 × SSC, and 0.1% SDS.

Herein, the nucleotide sequence of a 16S rRNA gene can be analyzed, for example, by the following method. First, a genomic DNA is extracted from a target microorganism to amplify the 16S rRNA gene using a known method. The method for amplifying the 16S rRNA gene is not particularly limited. Examples thereof include a PCR method using a universal primer commonly used by those skilled in the art. The amplified product obtained by the PCR method can be purified, as required, and subjected to a DNA sequencer or the like, to determine the nucleotide sequence. The nucleotide sequence obtained is compared with the sequence of SEQ ID No: 1.

Herein, the 16S rRNA gene is preferably an endogenous 16S rRNA gene originally possessed by the bacteria but may be an artificially mutated 16S rRNA gene. The mutation in the 16S rRNA gene is a mutation in which the expression and function of 16S rRNA are not lost.

Bacteria a have the ability to assimilate mannitol. The ability to assimilate mannitol can be determined based on a sugar assimilability test that is commonly performed in a microbial identification test. For example, the target microorganism is cultured in a medium that contains mannitol in an anaerobic environment, and it can be determined that the microorganism have the ability to assimilate mannitol if fermentation occurs. For the sugar assimilability test, a commercially available kit may be used. API50CHB (available from bioMerieux S.A., France) can be used, for example.

Bacteria a have the ability to control a plant disease. The ability to control a plant disease includes the ability to prevent infection of a plant with a pathogenic microorganism, prevent a plant disease, prevent expansion of a plant disease, kill and decompose a disease microorganism, and inhibit growth thereof.

Herein, the plant disease is, for example, a plant disease caused by *Ralstonia solanacearum* or *Ralstoniapseudosolanacearum.* The plant infected with *Ralstonia solanacearum* or *Ralstoniapseudosolanacearum* develops bacterial wilt or damping-off to wither.

Whether or not the target microorganism has the ability to control a plant disease can be examined, for example, by a method involving culturing a disease microorganism in the presence of the target microorganism or a method involving culturing a disease microorganism in the presence of a cell culture product of the target microorganism. In the case where the disease microorganism is killed or inhibited from growth under the aforementioned conditions, it can be determined that the target microorganism have the ability to control a plant disease. Also in the case where in cultivating a plant in the presence of the pathogen of a plant disease, the infection of the plant with the disease microorganism or onset of the disease is inhibited by the target microorganism, it can be determined that the target microorganism have the ability to control a plant disease.

Bacteria b according to one embodiment of the present invention comprise a 16S rRNA gene that contains a nucleotide sequence with an identity of 99.90 % or more to the nucleotide sequence of SEQ ID No: 1 and have the ability to control a plant disease. Bacteria b may include a 16S rRNA gene that contains the nucleotide sequence of SEQ ID No: 1. Examples of the bacteria that comprise a 16S rRNA gene that contains a nucleotide sequence with an identity of 99.90 % or more to the nucleotide sequence of SEQ ID No: 1 include bacteria belonging to the genus *Lactobacillus* (lactic acid bacteria).

Bacteria b may include a 16S rRNA gene that contains a nucleotide sequence with one base deleted or added in the nucleotide sequence of SEQ ID No: 1.

Bacteria b may include a 16S rRNA gene that contains a continuous sequence of about 15 bases or more, preferably about 18 to about 500 bases, more preferably about 18 to about 200 bases, further preferably about 18 to about 50 bases contained in the nucleotide sequence of SEQ ID No: 1 or a nucleotide sequence that hybridizes to a complementary sequence thereof under stringent conditions. The stringent conditions refer to conditions in which a non-specific hybrid is not formed. Examples thereof include conditions in which washing is performed one or more times in 60°C, $1 \times$ SSC, and 0.1% SDS, preferably 68°C, $0.1 \times$ SSC, and 0.1% SDS.

Bacteria b have the ability to control a plant disease. The plant disease is, for example, a plant disease caused by *Ralstonia solanacearum* or *Ralstonia pseudosolanacearum.* The ability to control a plant disease can be evaluated by the aforementioned methods.

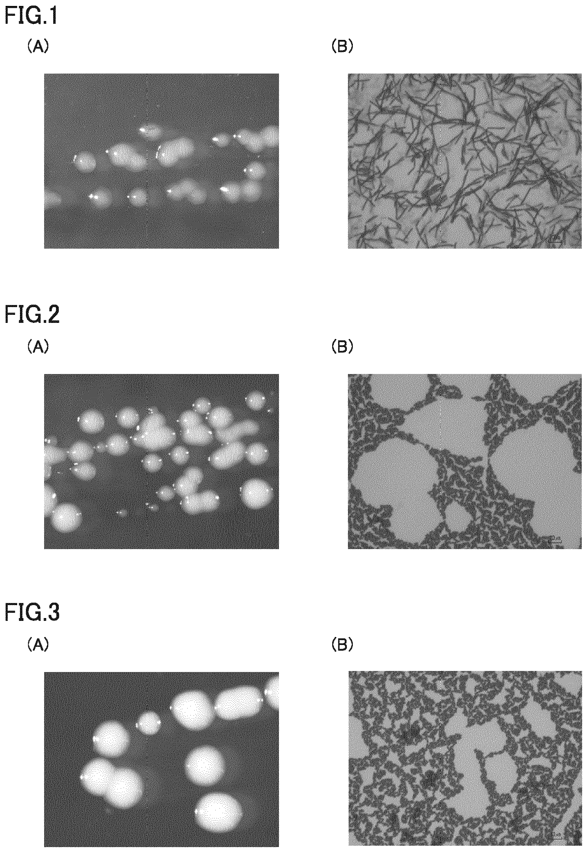

Typical examples of bacteria a and bacteria b include *Lactobacillus* sp. SC-2001. *Lactobacillus* sp. SC-2001 is a new separated strain of the genus *Lactobacillus* closely related to *Lactobacillus mali. Lactobacillus* sp. SC-2001 has been internationally deposited in the National Institute of Technology and Evaluation NITE Patent Microorganisms Depositary (NPMD, Address: Room 122, 2-5-8, Kazusakamatari, Kisarazu, Chiba, Japan 292-0818) with Accession Number: NITE BP-03197 (Original date of deposit: April 9, 2020) based on the Budapest Treaty. Table 1, Table 2, and FIG. 1 below show the mycological properties of the strain.

The lactic acid bacteria of the genus *Lactobacillus* including bacteria a and bacteria b are considered to be highly safe as food and feed since they exist in the natural environment. Bacteria a and bacteria b may be isolated bacteria. Bacteria a and bacteria b may be the same bacteria.

Bacteria c according to one embodiment of the present invention may be any one of bacteria internationally deposited in the National Institute of Technology and Evaluation NITE Patent Microorganisms Depositary (NPMD, Address: Room 122, 2-5-8, Kazusakamatari, Kisarazu, Chiba, Japan 292-0818) with Accession Number: NITE BP-03198 (Original date of deposit: April 9, 2020), Accession Number: NITE BP-03199 (Original date of deposit: April 9, 2020), Accession Number: NITE BP-03200 (Original date of deposit: April 9, 2020), Accession Number: NITE BP-03201 (Original date of deposit: April 9, 2020), and Accession Number: NITE BP-03202 (Original date of deposit: April 9, 2020) based on the Budapest Treaty. All the examples of bacteria c are bacteria belonging to *Lactobacillus plantarum.* Table 3 to Table 12 and FIG. 2 to FIG. 6 below show the mycological properties of the strain.

Bacteria c have the ability to control a plant disease. The plant disease is, for example, a plant disease caused by *Ralstonia solanacearum* or *Ralstonia pseudosolanacearum.* The ability to control a plant disease can be evaluated by the aforementioned methods. Further, *Lactobacillus plantarum* to which bacteria c belong is considered to have the ability to control a plant disease caused by *Ralstonia solanacearum* or *Ralstonia pseudosolanacearum.*

The lactic acid bacteria of the genus *Lactobacillus* including bacteria c are considered to be highly safe as food and feed since they exist in the natural environment. Bacteria c may be isolated bacteria.

[Bacterial cells or a cell culture product or extract thereof]

Bacterial cells according to one embodiment of the present invention are bacterial cells of any one of bacteria a, bacteria b, and bacteria c. The bacterial cells may be isolated from the environment or cultured. The bacterial cells may be dead bacteria or live bacteria. The bacterial cells may be present in a culture solution, a buffer, or the like, or may be a product obtained by concentrating them and removing the liquid or a freeze-dried product thereof. The bacterial cells of bacteria a, bacteria b, and bacteria c each have the ability to control a plant disease.

A cell culture product according to one embodiment of the present invention is a cell culture product of any one of bacteria a, bacteria b, and bacteria c. The cell culture product includes a secretion, a metabolite, or the like of bacteria, including a peptide, a protein, sugar, an enzyme, and an organic acid produced in bacteria and a medium containing these (liquid medium and solid medium). The cell culture product may be a supernatant obtained by culturing bacteria. The culture supernatant can be obtained, for example, by removing bacteria from a liquid medium used for culturing the bacteria by centrifugation, filtration, or the like. The cell culture products of bacteria a, bacteria b, and bacteria c each have the ability to control a plant disease.

Bacteria a, bacteria b, and bacteria c can be cultured according to a common culture method in bacteria belonging to the genus *Lactobacillus.* Typical examples of the method include a method involving culturing bacteria using a MRS (de Man, Rogosa and Sharpe) liquid medium or an MRS agar medium at a temperature of 30°C.

An extract according to one embodiment of the present invention is the extract of the bacterial cells or the cell culture product of any one of bacteria a, bacteria b, and bacteria c. The extract is prepared so that the ability to control a plant disease of the bacterial cells or the cell culture product is not lost. The extract can be obtained, for example, by subjecting the bacterial cells or the cell culture product to treatment such as ultrasonic crushing, bead grinding, freezing and thawing, and chemical dissolution. The extract may be obtained by subjecting the bacterial cells or the cell culture product to salting-out, ultrafiltration, ion-exchange chromatography, or liquid phase extraction using an organic solvent. These treatments can be appropriately combined. The extract can contain a fragment of the bacterial cells, a nucleic acid, a peptide, a protein, or an enzyme.

[control agent and control method against a plant disease caused by *Ralstonia solanacearum* or *Ralstonia pseudosolanacearum*]

Herein, the bacterial cells or the cell culture product, or the extract thereof are also referred to as "bacterial cell preparation". The bacterial cell preparation may be any one selected from the group consisting of the bacterial cells, the cell culture product, and the extract thereof or may be two or more of them. A control agent according to one embodiment of the present invention contains a bacterial cell preparation of any one of bacteria a to c. A control agent according to one embodiment of the present invention contains a bacterial cell preparation of bacteria belonging to *Lactobacillus plantarum* (which may be hereinafter referred to as "bacteria d"). The control agent may contain bacterial cell preparations of two or more selected from the group consisting of bacteria a to d. The control agent according to the present invention has the ability to control a plant disease caused by *Ralstonia solanacearum* or *Ralstoniapseudosolanacearum.* The control ability can be evaluated by the aforementioned methods.

The control agent may be in solid, liquid, or gaseous form that common agricultural chemicals can be in, and examples of the form include, but not specifically limited to, a powder formulation, a DL (Driftless) powder formulation, a granule, a tablet, a hydrating agent, a granular hydrating agent, a dry flowable, a cream, a liquid formulation, an oil agent, a microcapsule, a flowable, an emulsion, a microemulsion, an AL (applicable liquid) agent, and a fumigant. The control agent can be prepared and used in any dosage form according to the purpose.

The control agent may contain a carrier, an aggregation inhibitor, a decomposition inhibitor, an extender, a dispersant, a binding material, a disintegrant, a surfactant for formulation. As a solid carrier, diatomite, vermiculite, clay, talc, bentonite, perlite, white carbon, chaff, bone meal, calcium carbonate, or the like can be used. Examples of a liquid carrier include water, alcohols, ketones (such as acetone, methyl ethyl ketone, and cyclohexanone), aromatic hydrocarbons (such as benzene, toluene, xylene, ethyl benzene, and methyl naphthalene), aliphatic hydrocarbons (such as n-hexane and kerosene), esters, nitriles, ethers, amides, and hydrocarbon halides. Examples of the gaseous carrier include LPG, air, nitrogen, carbon dioxide gas, and dimethyl ether.

As a surfactant or a dispersant, alkyl sulfate esters, alkyl (aryl) sulfonates, polyoxyalkylene alkyl (aryl) ethers, polyhydric alcohol esters, lignosulfonates, or the like can be used.

The control agent may contain a microorganism other than bacteria a to d, a chemical control substance, or the like, as long as the ability to control a plant disease caused by *Ralstonia solanacearum* or *Ralstoniapseudosolanacearum* is not lost.

The control agent according to the present invention has a small burden on the environment and is less toxic to a human. The control agent according to the present invention is considered to have a direct antibacterial activity against a microorganism with a plant disease regardless of the crop variety. The control agent according to the present invention enables an agricultural product to be produced stably.

One embodiment of the present invention is use of a bacterial cell preparation of at least one selected from the group consisting of bacteria a to d for production of a control agent against a plant disease caused by *Ralstonia solanacearum* or *Ralstonia pseudosolanacearum.*

A control method according to one embodiment of the present invention includes applying the aforementioned control agent. The control method according to the present invention enables a plant disease caused by *Ralstonia solanacearum* or *Ralstonia pseudosolanacearum* to be controlled.

Examples of applying the control agent include applying a liquid control agent to a plant. Applying a liquid control agent to a plant includes applying the control agent to a seed, such as spraying to a seed of a plant or immersing the seed. Applying a liquid control agent to a plant also includes immersing a seedling before planting, a root of a plant that has grown from a seedling, or the like in the control agent and spraying the control agent to a root, a leaf, a stem, or the like.

Other examples of applying the control agent include applying the liquid control agent to a nutrient solution or soil. Applying the liquid control agent to a nutrient solution includes adding the control agent to the nutrient solution or diluting the control agent with the nutrient solution. Applying the liquid control agent to soil includes spraying, spreading, or irrigating the control agent to soil. Applying the control agent to soil may be performed before planting or before seeding or may be performed after planting or after seeding.

Other examples of applying the control agent include applying a powder or granular control agent to a plant. Applying the powder or granular control agent to a plant includes attaching the powder or granular control agent to a seed surface, mixing and seeding a seed with the powder or granular control agent, and applying the powder or granular control agent to a seed.

Other examples of applying the control agent include applying the powder or granular control agent to a nutrient solution or soil. Applying the powder or granular control agent to a nutrient solution includes spreading or dispersing the control agent to a nutrient solution. Applying the powder or granular control agent to soil includes mixing the powder or granular control agent with soil, spreading the powder or granular control agent on soil, or the like.

A solid or liquid control agent may be suspended or diluted with water or a nutrient solution for application. When applying the control agent to a nutrient solution, the control agent preferably includes bacterial cells and/or a cell culture product of at least one selected from the group consisting of bacteria a to d. When applying the control agent to soil, the control agent preferably includes the bacterial cells and/or cell culture product of at least one selected from the group consisting of bacteria a to d, more preferably live bacteria of at least one selected from the group consisting of bacteria a to d. The application of the control agent may be performed multiple times, and the plurality of steps described above may be combined.

Applying the control agent may be performed under conditions in which the control ability is not lost. For example, the temperature may be 20 to 40°C or 25 to 30°C. Further, the pH may be 3.0 to 8.0 or 4.0 to 6.0.

The total concentration of bacteria a to d in the control agent is not specifically limited and may be $1 \times 10^3$ to $1 \times 10^{12}$ cfu (colony forming unit)/g, $1 \times 10^4$ to $1 \times 10^{12}$ cfu/g, or $1 \times 10^5$ to $1 \times 10^{12}$ cfu/g. The concentration of the cell culture product or extract in the control agent is not specifically limited and may be 0.1 to 100,000 ppm, 1 to 100,000 ppm, or 10 to 100,000 ppm. The control agent, for example, may be applied by culturing at least one selected from the group consisting of bacteria a to d to give a cell culture supernatant (cell culture product), preparing a solution of the supernatant with water to the above concentration, and mixing 0.01 to 100 mL of the solution per 50 g of soil. The control agent, for example, may be applied by culturing at least one selected from the group consisting of bacteria a to d to give a supernatant, and mixing it with a nutrient solution at 1/10000 to 1/10. The control agent may be applied in an amount suitable for the weather conditions, the formulation form, the application time, the application method, the application place, the target plant, or the like.

Herein, the plant is, for example, a plant in which a disease may be caused by *Ralstonia solanacearum* or *Ralstonia pseudosolanacearum*. Such a plant is, for example, an agricultural and horticultural plant, and specific examples thereof include a *Solanaceae* plant, a *Cucurbitaceae* plant, a *Brassicaceae* plant, a *Zingiberaceae* plant, a *Rosaceae* plant, an *Asteraceae* plant, a *Leguminosae* plant, a *Musaceae* plant, a *Labiatae* plant, a *Myrtaceae* plant, a *Pedaliaceae* plant, a *Moraceae* plant, a *Strelitziaceae* plant, a *Euphorbiaceae* plant, a *Plumbaginaceae* plant, and a *Gentianaceae* plant. Examples of the *Solanaceae* plant include tomato, eggplant, green pepper, sweet green pepper, capsicum, paprika, potato, and tobacco. Examples of the *Cucurbitaceae* plant include cucumber, pumpkin, bitter gourd, watermelon, melon, and zucchini. Examples of the *Brassicaceae* plant include radish, turnip, rape, ching guang juai, brassica campestris, cauliflower, broccoli, and a kale. Examples of the *Zingiberaceae* plant include ginger and Japanese ginger. Examples of the *Rosaceae* plant include strawberry. Examples of the *Asteraceae* plant include garland chrysanthemum and lettuce. Examples of the *Leguminosae* plant include peanut, common bean, broad bean, pea, and soybean. Examples of the *Musaceae* plant include banana and Musa basjoo. Examples of the *Labiatae* plant include *Perilla* and basil. Examples of the *Myrtaceae* plant include clove. Examples of the *Pedaliaceae* plant include sesame seed. Examples of the *Moraceae* plant include mulberry. Examples of the *Strelitziaceae* plant include strelitzia. Examples of the *Euphorbiaceae* plant include cassava. Examples of the *Plumbaginaceae* plant include statice. Examples of the *Gentianaceae* plant include Lisianthus. As specific examples of the plant species, plants for crops are mainly mentioned above, but the plant may be an ornamental plant.

One embodiment of the present invention is use of the control agent for controlling a plant disease caused by *Ralstonia solanacearum* or *Ralstonia pseudosolanacearum*. One embodiment of the present invention is use of a bacterial cell preparation of at least one selected from the group consisting of bacteria a to d for controlling a plant disease caused by *Ralstonia solanacearum* or *Ralstonia pseudosolanacearum*.

[Disinfectant and disinfection method for plant, nutrient solution, soil, nutriculture material, or soil culture material contaminated by *Ralstonia solanacearum* or *Ralstonia pseudosolanacearum*]

A disinfectant according to one embodiment of the present invention contains a bacterial cell preparation of any one of bacteria a to c. A disinfectant according to one embodiment of the present invention contains a bacterial cell preparation of bacteria d. The disinfectant may contain a bacterial cell preparation of two or more selected from the group consisting of bacteria a to d. The disinfectant according to the present invention can disinfect a plant, a nutrient solution, soil, a nutriculture material, or a soil culture material contaminated by *Ralstonia solanacearum* or *Ralstonia*

*pseudosolanacearum.* When a plant is cultivated using the disinfected plant, nutrient solution, soil, nutriculture material, or soil culture material, the development of a plant disease caused by *Ralstonia solanacearum* or *Ralstoniapseudosolanacearum* can be inhibited. The disinfectant according to the present invention also has an effect as a bactericide against *Ralstonia solanacearum* or *Ralstoniapseudosolanacearum.*

The effect of the disinfectant can be evaluated by a method involving culturing *Ralstonia solanacearum* or *Ralstonia pseudosolanacearum* in the presence of the disinfectant according to the present invention and examining whether or not *Ralstonia solanacearum* or *Ralstoniapseudosolanacearum* is killed, decomposed, or inhibited from growth.

The control agent as aforementioned can be used as the disinfectant. The disinfectant may contain a substance used for a disinfection for a microorganism with another disease, or the like.

The plant includes a seed, a seedling, a root, a tuber, a bulb, a rhizome, a leave, and a stem (including a flower). For preventing infection with disease bacteria, disinfection of the seedling, root, or the like of the plant is particularly useful.

Nutriculture is a cultivation method in which nutrient and water necessary for growth is given to the plant as a liquid fertilizer (nutrient solution) without using soil. Typical examples of nutriculture include hydroponics involving growing a root in a nutrient solution, solid medium cultivation involving planting a crop in a solid medium instead of soil, and spraying hydroponics involving spraying a nutrient solution to a root. The nutrient solution contains nitrogen (N), phosphoric acid ($P_2O_5$), potassium ($K_2O$), or the like. Examples of the nutriculture material used for nutriculture include a cultivation bed, a cultivation sheet, a planting panel, a seedling pot, a solid medium (such as rockwool), a nutrient solution bath, a nutrient solution tube, a watering tube, and a small farm tool (such as pruning shears, a thermometer, and a hygrometer).

Soil culture is a cultivation method using soil. Soil includes cultivation soil for sowing, cultivation soil for raising seedlings, sand, pumice, field soil, or the like. Examples of the soil culture material include a planter, a seedling pot, a cultivation sheet, a cultivation container, a spreader, a watering tube, and a small farm tool (such as pruning shears, a thermometer, and a hygrometer).

One embodiment of the present invention is use of a bacterial cell preparation of at least one selected from the group consisting of bacteria a to d for production of a disinfectant or a bactericide for a plant, a nutrient solution, soil, a nutriculture material, or a soil culture material contaminated by *Ralstonia solanacearum* or *Ralstonia pseudosolanacearum.*

A disinfection method according to one embodiment of the present invention includes using the aforementioned disinfectant. The disinfection method according to the present invention enables a plant, a nutrient solution, soil, a nutriculture material, and a soil culture material contaminated by *Ralstonia solanacearum* or *Ralstonia pseudosolanacearum* to be disinfected.

The disinfectant may be used for a plant, a nutrient solution, soil, a nutriculture material, or a soil culture material that is contaminated or may be contaminated by *Ralstonia solanacearum* or *Ralstoniapseudosolanacearum.* In the method for disinfecting soil, a nutrient solution, or a plant, using a disinfectant may be performed in the same manner as for applying the control agent described above. In the method for disinfecting a nutriculture material or a soil culture material, using a disinfectant includes spraying or spreading a liquid disinfectant or a diluted or suspended disinfectant onto the nutriculture material or the soil culture material, or immersing it in the disinfectant, for example. Using a disinfectant may be performed simultaneously with a known disinfection method for a plant, a nutrient solution, soil, a nutriculture material, or a soil culture material such as a soil fumigation method.

The total concentration of bacteria a to d in the disinfectant is not specifically limited and may be $1 \times 10^3$ to $1 \times 10^{12}$ cfu/g, $1 \times 10^4$ to $1 \times 10^{12}$ cfu/g, $1 \times 10^5$ to $1 \times 10^{12}$ cfu/g. The concentration of the cell culture product or extract in the disinfectant is not specifically limited and may be 0.1 to 100,000 ppm, 1 to 100,000 ppm, 10 to 100,000 ppm. The disinfectant may be used in an amount suitable for the weather conditions, the formulation form, the use time, the use method, the use place, the target plant, or the like.

One embodiment of the present invention is use of the aforementioned disinfectant for disinfecting or sterilizing a plant, a nutrient solution, soil, a nutriculture material, or a soil culture material contaminated by *Ralstonia solanacearum* or *Ralstonia pseudosolanacearum.* One embodiment of the present invention is use of a bacterial cell preparation of at least one selected from the group consisting of bacteria a to d for disinfecting or sterilizing a plant, a nutrient solution, soil, a nutriculture material, or a soil culture material contaminated by *Ralstonia solanacearum* or *Ralstonia pseudosolanacearum.*

[Growth inhibitor and growth-inhibiting method for *Ralstonia solanacearum* or *Ralstonia pseudosolanacearum*]

A growth inhibitor according to one embodiment of the present invention includes a bacterial cell preparation of any one of bacteria a to c. A growth inhibitor according to one embodiment of the present invention includes a bacterial cell preparation of bacteria d. The growth inhibitor may contain bacterial cell preparations of two or more selected from the group consisting of bacteria a to d. The growth inhibitor according to the present invention can inhibit the growth of *Ralstonia solanacearum* or *Ralstoniapseudosolanacearum.* When the growth of *Ralstonia solanacearum* or *Ralstoni-*

*apseudosolanacearum* is inhibited, development of a plant disease caused thereby is inhibited.

The control agent as aforementioned can be used as the growth inhibitor. The growth inhibitor may contain a substance or the like that inhibits the growth of a microorganism with another disease.

The effect of the growth inhibitor can be evaluated by a method involving culturing *Ralstonia solanacearum* or *Ralstoniapseudosolanacearum* in the presence of the growth inhibitor and examining whether or not the growth of *Ralstonia solanacearum* or *Ralstoniapseudosolanacearum* is inhibited.

One embodiment of the present invention is use of a bacterial cell preparation of at least one selected from the group consisting of bacteria a to d for production of the growth inhibitor for *Ralstonia solanacearum* or *Ralstoniapseudosolanacearum.*

A growth-inhibiting method according to one embodiment of the present invention includes using the aforementioned growth inhibitor. The growth-inhibiting method according to the present invention can inhibit the growth of *Ralstonia solanacearum* or *Ralstoniapseudosolanacearum* and the development of a plant disease caused by *Ralstonia solanacearum* or *Ralstonia pseudosolanacearum.*

Using the growth inhibitor is not specifically limited, and the growth inhibitor may be used so that the growth inhibitor comes in contact with or close to *Ralstonia solanacearum* or *Ralstoniapseudosolanacearum.* The growth inhibitor may be used by the same method as for the aforementioned control agent.

The total concentration of bacteria a to d in the growth inhibitor is not specifically limited and may be $1 \times 10^3$ to $1 \times 10^{12}$ cfu/g, $1 \times 10^4$ to $1 \times 10^{12}$ cfu/g, $1 \times 10^5$ to $1 \times 10^{12}$ cfu/g. The concentration of the cell culture product or extract in the growth inhibitor is not specifically limited and may be 0.1 to 100,000 ppm, 1 to 100,000 ppm, 10 to 100,000 ppm.

One embodiment of the present invention is use of the aforementioned growth inhibitor for inhibiting the growth of *Ralstonia solanacearum* or *Ralstonia pseudosolanacearum.* One embodiment of the present invention is use of a bacterial cell preparation of at least one selected from the group consisting of bacteria a to d for inhibiting the growth of *Ralstonia solanacearum* or *Ralstoniapseudosolanacearum.*

[Method for cultivating plant]

A cultivation method according to one embodiment of the present invention plant includes cultivating a plant in a nutrient solution or soil containing a bacterial cell preparation of at least one bacteria selected from the group consisting of bacteria a to c. The method for cultivating a plant according to the present invention can inhibit a plant disease caused by *Ralstonia solanacearum* or *Ralstonia pseudosolanacearum.*

The method for incorporating, into a nutrient solution or soil, a bacterial cell preparation of at least one bacteria selected from the group consisting of bacteria a to c is not specifically limited, and the same method as the method for applying the control agent can be used. The bacterial cell preparation of at least one bacteria selected from the group consisting of bacteria a to c can be contained as the aforementioned control agent in the nutrient solution or soil.

The method for cultivating a plant is nutriculture or soil cultivation. The method for cultivating a plant may include providing a nutrient solution or soil, seeding, irradiation with light, thinning out, and growing the plant, for example. A nutrient solution or soil containing a bacterial cell preparation of at least one bacteria selected from the group consisting of bacteria a to c may be used in at least one of these steps or all the steps.

One embodiment of the present invention is use of the aforementioned control agent in a method for cultivating a plant in a nutrient solution or soil. One embodiment of the present invention is use of a bacterial cell preparation of at least one selected from the group consisting of bacteria a to d in a method for cultivating a plant in a nutrient solution or soil.

[Examples]

Hereinafter, the present invention will be described further in detail by way of examples, but the present invention is not limited to these examples.

[Experiment 1: Separation of new microorganism]

A separation source (*Quercus serrata*) was ground together with sterile water. The ground solution was appropriately diluted and added to a 1/2 MRS liquid medium, followed by enrichment culture. The enrichment culture solution was smeared on an MRS agar medium containing calcium carbonate, and a microorganism that formed halo was separated. Hereinafter, this separated strain is referred to as a separated strain A. The culture solution of separated strain A was suspended in hydrogen peroxide, and no bubble was generated. Separated strain A was confirmed to be lactic acid bacteria since it did not have catalase activity.

[Experiment 2: Identification of separated strain A]

Separated strain A was identified by the 16S rRNA gene analysis, the morphological observation, and the physiological and biochemical characterization test.

(1) 16S rRNA gene analysis

A genomic DNA was extracted from separated strain A, and the 16S rRNA gene was amplified by PCR using the genomic DNA obtained as a template, and a forward primer 9F for cloning and a reverse primer 1510R for cloning (NAKAGAWA Yasuyoshi et al.: Gene analysis method, 16S rRNA gene sequencing, edited by the Society for Actinomycetes Japan, Classification and identification of actinomycetes, 88-117 pp., the Center for Academic Societies Japan, 2001). PCR amplification was performed using Tks Gflex DNA polymerase (available from Takara Bio Inc.), and the amplified product after PCR was purified.

A cycle sequence reaction was performed on the amplified product purified after PCR. The cycle sequence reaction was performed using a BigDye Terminator v3.1 Cycle Sequencing Kit. The reaction solution obtained was purified, and the purified solution was subjected to DNA sequence analysis (3130xl DNA Analyzer), to determine the nucleotide sequence of the 16S rRNA gene of the template DNA extracted from separated strain A (SEQ ID No: 1). As primers for sequence analysis, 9F, 515F, 1099F, 536R, 926R, and 1510R (NAKAGAWA Yasuyoshi et al.: Gene analysis method, 16S rRNA gene sequencing, edited by the Society for Actinomycetes Japan, Classification and identification of actinomycetes, 88-117 pp., the Center for Academic Societies Japan, 2001) were used.

Using a microorganism identifying system "ENKI" (available from TechnoSuruga Laboratory Co., Ltd.), the nucleotide sequence of the 16S rRNA gene of separated strain A was subjected to BLAST homology search on a microorganism identifying database DB-BA15.0 (available from TechnoSuruga Laboratory Co., Ltd.) and an international nucleotide sequence database (DDBJ/ENA(EMBL)/GenBank). As a result, the nucleotide sequence showed an identity of 99.87% to the nucleotide sequence of the 16S rRNA gene of *Lactobacillus mali* (NBRC 102159), an identity of 98.72% to the nucleotide sequence of the 16S rRNA gene of *Lactobacillus cacaonum* (LMG24285), and an identity of 97.58% to the nucleotide sequence of the 16S rRNA gene of *Lactobacillus aquatius* (IMCC1736). However, no microorganism had a 16S rRNA gene that completely matched the nucleotide sequence of the 16S rRNA gene of separated strain A.

(2) Morphological observation and physiological and biochemical characterization test

Separation bacteria A was applied to an MRS agar medium, followed by aerobic culture at a temperature of 30°C for 48 hours, to observe the cell morphology, gram stainability, motility, and colony morphology by the following methods. Using an API50CHB kit (available from bioMerieux S.A., France), the physiological and biochemical characteristic reactions of bacteria were examined.

Colony observation with a stereomicroscope SMZ800N (available from NIKON CORPORATION), morphological observation with an optical microscope BX50F4 (available from Olympus Corporation), and the tests of catalase reaction, oxidase reaction, acid/gas production from glucose and oxidation/fermentation (O/F) of glucose by the method according to Barrow&Feltham (Cowan and Steel's Manual for the Identification of Medical Bacteria, 3rd ed. Cambridge: Cambridge University Press; 1993.) were performed. For gram staining, Favor G "NISSUI" (available from Nissui Pharmaceutical Co., Ltd.) was used. As shown in FIG. 1(A), separated strain A formed a circular colony. As shown in FIG. 1(B), separated strain A was positive for gram stainability. Table 1 and Table 2 show the results of a physiological and biochemical characterization test and a fermentability test for separated strain A. Separated strain A was a gram-positive rod with motility that fermented glucose and was negative for catalase reaction and oxidase reaction. These characteristics matched the characteristics of the genus *Lactobacillus,* to which attribution was shown as a result of a 16S rDNA partial nucleotide sequence analysis. As a result of a fermentability test using an API CHL50 kit, separated strain A fermented fructose, mannose, mannitol, salicin, and the like and did not ferment galactose, lactose, and the like. Further, separated strain A grew at 15°C and did not exhibit arginine dihydrolase activity. These characteristics mostly matched the characteristics of *L. mali,* which was suggested to be closely related as a result of a 16S rDNA partial nucleotide sequence analysis, but were different in that mannitol was fermented (Hammes WP and Hertel C.Lactobacillus. In: Whitman WB, Rainey F, Kampfer P, Trujillo M, Chun J et al. (editors). Bergey's Manual of Systematics of Archaea and Bacteria. Chichester: John Wiley & Sons; 2015. doi: 10.1002/9781118960608.gbm00604). As described above, separated strain A was included in the genus *Lactobacillus* and most closely related to *L. mali* among known species, but it turned out to be a new separated strain closely related to *Lactobacillus mali* since the results of the 16S rDNA nucleotide sequence analysis and the physiological and biochemical characteristics test suggested that separated strain A was slightly different from *L. mali.* Separated strain A was named *Lactobacillus* sp. SC-2001 and internationally deposited as NITE BP-03197.

[Table 1]

| Test item | | NITE BP-03197 |
|---|---|---|
| Culture temperature | | 30°C |
| Cell morphology | | Rod (0.7-0.8×5.0-10.0 μm) |
| Gram stainability | | + |
| Presence or absence of spore | | - |
| Motility | | + |
| Colony morphology | Medium | MRS agar medium |
| | Culture time | 48 hours |
| | Diameter | 1 to 2 mm |
| | Color Tones | White |
| | Shape | Circular |
| | Raised state | Lens shape |
| | Circumferential edge | Entire edge |
| | Surface shape, etc. | Smooth |
| | Transparency | Opaque |
| | Consistency | Butter-like |
| Growth temperature test | 15°C | + |
| | 37°C | + |
| | 45°C | - |
| Arginine dihydrolase activity | | - |
| Catalase reaction | | - |
| Oxidase reaction | | - |
| Acid/gas production from glucose (Acid production/gas production) | | + / - |
| O/F test (Oxidation/Fermentation) | | + / + |
| + : Positive, - : Negative | | |

[Table 2]

| Item | Substrate component | Test results | Item | Substrate component | Test results |
|---|---|---|---|---|---|
| 0 | Control | - | 25 | Esculin | + |
| 1 | Glycerol | - | 26 | Salicin | + |
| 2 | Erythritol | - | 27 | Cellobiose | + |
| 3 | D-Arabinose | - | 28 | Maltose | - |
| 4 | L-Arabinose | - | 29 | Lactose | - |
| 5 | Ribose | - | 30 | Melibiose | - |
| 6 | D-Xylose | - | 31 | Sucrose | + |
| 7 | L-Xylose | - | 32 | Trehalose | + |
| 8 | Adonitol | - | 33 | Inulin | - |

(continued)

| Item | Substrate component | Test results | Item | Substrate component | Test results |
|------|---------------------|--------------|------|---------------------|--------------|
| 9 | β-Methyl-D-xylose | - | 34 | Melezitose | - |
| 10 | Galactose | - | 35 | Raffinose | - |
| 11 | Glucose | + | 36 | Starch | - |
| 12 | Fructose | + | 37 | Glycogen | - |
| 13 | Mannose | + | 38 | Xylitol | - |
| 14 | Sorbose | - | 39 | Gentiobiose | - |
| 15 | Rhamnose | - | 40 | D-Turanose | - |
| 16 | Dulcitol | - | 41 | D-Lyxose | - |
| 17 | Inositol | - | 42 | D-Tagatose | + |
| 18 | Mannitol | + | 43 | D-Fucose | - |
| 19 | Sorbitol | - | 44 | L-Fucose | - |
| 20 | α-Methyl-D-mannoside | + | 45 | D-Arabitol | - |
| 21 | α-Methyl-D-glucoside | - | 46 | L-Arabitol | - |
| 22 | N-Acetylglucosamine | + | 47 | Gluconate | - |
| 23 | Amygdalin | + | 48 | 2-Ketogluconate | - |
| 24 | Arbutin | + | 49 | 5-Ketogluconate | - |
| + : Positive, - : Negative | | | | | |

[Experiment 3: Separation and identification of separated strain B]

Separated strain B was separated in the same manner as in Experiment 1 except that salted squid was used as a separation source. Identification of separated strain B was performed in the same manner as in Experiment 2.

The nucleotide sequence of the 16S rRNA gene of separated strain B showed an identity of 99.87% to the nucleotide sequence of the 16S rRNA gene of *Lactobacillus pentosus* (JCM1558), an identity of 99.87% to the nucleotide sequence of the 16S rRNA gene of *Lactobacillus plantarum* subsp. *plantarum* (JCM1149), and an identity of 99.73% to the nucleotide sequence of the 16S rRNA gene of *Lactobacillus paraplantarum* (DSM10667). However, no microorganism had a 16S rRNA gene that completely matched the nucleotide sequence of the 16S rRNA gene of separated strain B.

As shown in FIG. 2(A), separated strain B formed a circular colony. As shown in FIG. 2(B), separated strain B was positive for gram stainability. Table 3 and Table 4 show the results of a physiological and biochemical characterization test and a fermentability test for separated strain B. Separated strain B was a gram-positive rod without motility that did not form a spore, was negative for catalase reaction and oxidase reaction, and fermented glucose. These characteristics matched the characteristics of the genus *Lactobacillus,* to which the possibility of attribution was shown as a result of a 16S rDNA partial nucleotide sequence analysis. As a result of a fermentability test using an API kit, separated strain B fermented galactose, fructose, melezitose, and the like and did not ferment glycerol, D-xylose, and the like. Further, separated strain B did not exhibit arginine dihydrolase activity and grew at 15°C. For *L. pentosus* and *L. plantarum,* to which attribution was suggested as a result of a 16S rDNA partial nucleotide sequence analysis, these characteristics matched the characteristics of *L. plantarum* while being different from the characteristics of *L. pentosus* in that glycerol or D-xylose were not fermented. Accordingly, separated strain B turned out to be a new separated strain belonging to *Lactobacillus plantarum.* Separated strain B was internationally deposited as NITE BP-03198.

[Table 3]

| Test item | NITE BP-03198 |
|-----------|----------------|
| Culture temperature | 30°C |
| Cell morphology | Rod (0.6-0.7×1.0-3.0 μm) |
| Gram stainability | + |

(continued)

| | | |
|---|---|---|
| Presence or absence of spore | | - |
| Motility | | - |
| Colony morphology | Medium | MRS agar medium |
| | Culture time | 48 hours |
| | Diameter | 1 to 2 mm |
| | Color Tones | Milky |
| | Shape | Circular |
| | Raised state | Lens shape |
| | Circumferential edge | Entire edge |
| | Surface shape, etc. | Smooth |
| | Transparency | Opaque |
| | Consistency | Butter-like |
| Growth temperature test | 15°C | + |
| | 37°C | + |
| | 45°C | - |
| Arginine dihydrolase activity | | - |
| Catalase reaction | | - |
| Oxidase reaction | | - |
| Acid/gas production from glucose (Acid production/gas production) | | + / - |
| O/F test (Oxidation/Fermentation) | | + / + |
| + : Positive, - : Negative | | |

[Table 4]

| Item | Substrate component | Test results | Item | Substrate component | Test results |
|---|---|---|---|---|---|
| 0 | Control | - | 25 | Esculin | + |
| 1 | Glycerol | - | 26 | Salicin | + |
| 2 | Erythritol | - | 27 | Cellobiose | + |
| 3 | D-Arabinose | - | 28 | Maltose | + |
| 4 | L-Arabinose | + | 29 | Lactose | + |
| 5 | Ribose | + | 30 | Melibiose | + |
| 6 | D-Xylose | - | 31 | Sucrose | + |
| 7 | L-Xylose | - | 32 | Trehalose | + |
| 8 | Adonitol | - | 33 | Inulin | - |
| 9 | β-Methyl-D-xylose | - | 34 | Melezitose | + |
| 10 | Galactose | + | 35 | Raffinose | + |
| 11 | Glucose | + | 36 | Starch | - |
| 12 | Fructose | + | 37 | Glycogen | - |
| 13 | Mannose | + | 38 | Xylitol | - |

(continued)

| Item | Substrate component | Test results | Item | Substrate component | Test results |
|------|---------------------|--------------|------|---------------------|--------------|
| 14 | Sorbose | - | 39 | Gentiobiose | + |
| 15 | Rhamnose | - | 40 | D-Turanose | + |
| 16 | Dulcitol | - | 41 | D-Lyxose | - |
| 17 | Inositol | - | 42 | D-Tagatose | - |
| 18 | Mannitol | + | 43 | D-Fucose | - |
| 19 | Sorbitol | + | 44 | L-Fucose | - |
| 20 | $\alpha$-Methyl-D-mannoside | + | 45 | D-Arabitol | - |
| 21 | $\alpha$-Methyl-D-glucoside | - | 46 | L-Arabitol | - |
| 22 | N-Acetylglucosamine | + | 47 | Gluconate | + |
| 23 | Amygdalin | + | 48 | 2-Ketogluconate | - |
| 24 | Arbutin | + | 49 | 5-Ketogluconate | - |
| + : Positive, - : Negative | | | | | |

[Experiment 4: Separation and identification of separated strain C]

Separated strain C was separated in the same manner as in Experiment 1 except that lianwu was used as a separation source. Identification of separated strain C was performed in the same manner as in Experiment 2.

The nucleotide sequence of the 16S rRNA gene of separated strain C showed an identity of 100.0% to the nucleotide sequence of the 16S rRNA gene of *Lactobacillus pentosus* (JCM1558), an identity of 100.0% to the nucleotide sequence of the 16S rRNA gene of *Lactobacillus plantarum* subsp. *plantarum* (JCM1149), and an identity of 99.80% to the nucleotide sequence of the 16S rRNA gene of *Lactobacillus paraplantarum* (DSM10667).

As shown in FIG. 3(A), separated strain C formed a circular colony. As shown in FIG. 3(B), separated strain C was positive for gram stainability. Table 5 and Table 6 show the results of a physiological and biochemical characterization test and a fermentability test for separated strain C. Separated strain C was a gram-positive rod without motility that did not form a spore, was negative for catalase reaction and oxidase reaction, and fermented glucose. These characteristics matched the characteristics of the genus *Lactobacillus,* to which the possibility of attribution was shown as a result of a 16S rDNA partial nucleotide sequence analysis. As a result of a fermentability test using an API kit, separated strain C fermented galactose, fructose, $\alpha$-methyl-D-mannoside and melezitose, and the like and did not ferment glycerol, D-xylose, and the like. Further, separated strain C did not exhibit arginine dihydrolase activity and grew at 15°C. For *L. pentosus* and *L. plantarum,* to which attribution was suggested as a result of a 16S rDNA partial nucleotide sequence analysis, these characteristics matched the characteristics of *L. plantarum* while being different from the characteristics of *L. pentosus* in that glycerol or D-xylose were not fermented. Accordingly, separated strain C turned out to be a new separated strain belonging to *Lactobacillus plantarum.* Separated strain C was internationally deposited as NITE BP-03199.

[Table 5]

| Test item | NITE BP-03199 |
|-----------|---------------|
| Culture temperature | 30°C |
| Cell morphology | Rod (0.7-0.8×2.0-3.0 μm) |
| Gram stainability | + |
| Presence or absence of spore | - |
| Motility | - |

(continued)

| Colony morphology | Medium | MRS agar medium |
|---|---|---|
| | Culture time | 48 hours |
| | Diameter | 1 to 3 mm |
| | Color Tones | Milky |
| | Shape | Circular |
| | Raised state | Lens shape |
| | Circumferential edge | Entire edge |
| | Surface shape, etc. | Smooth |
| | Transparency | Opaque |
| | Consistency | Butter-like |
| Growth temperature test | 15°C | + |
| | 37°C | + |
| | 45°C | - |
| Arginine dihydrolase activity | | - |
| Catalase reaction | | - |
| Oxidase reaction | | - |
| Acid/gas production from glucose (Acid production/gas production) | | + / - |
| O/F test (Oxidation/Fermentation) | | + / + |
| + : Positive, - : Negative | | |

[Table 6]

| Item | Substrate component | Test results | Item | Substrate component | Test results |
|---|---|---|---|---|---|
| 0 | Control | - | 25 | Esculin | + |
| 1 | Glycerol | - | 26 | Salicin | + |
| 2 | Erythritol | - | 27 | Cellobiose | + |
| 3 | D-Arabinose | - | 28 | Maltose | + |
| 4 | L-Arabinose | + | 29 | Lactose | + |
| 5 | Ribose | + | 30 | Melibiose | + |
| 6 | D-Xylose | - | 31 | Sucrose | + |
| 7 | L-Xylose | - | 32 | Trehalose | + |
| 8 | Adonitol | - | 33 | Inulin | - |
| 9 | β-Methyl-D-xylose | - | 34 | Melezitose | + |
| 10 | Galactose | + | 35 | Raffinose | + |
| 11 | Glucose | + | 36 | Starch | - |
| 12 | Fructose | + | 37 | Glycogen | - |
| 13 | Mannose | + | 38 | Xylitol | - |
| 14 | Sorbose | - | 39 | Gentiobiose | + |
| 15 | Rhamnose | - | 40 | D-Turanose | + |

(continued)

| Item | Substrate component | Test results | Item | Substrate component | Test results |
|---|---|---|---|---|---|
| 16 | Dulcitol | - | 41 | D-Lyxose | - |
| 17 | Inositol | - | 42 | D-Tagatose | - |
| 18 | Mannitol | + | 43 | D-Fucose | - |
| 19 | Sorbitol | + | 44 | L-Fucose | - |
| 20 | α-Methyl-D-mannoside | + | 45 | D-Arabitol | - |
| 21 | α-Methyl-D-glucoside | + | 46 | L-Arabitol | - |
| 22 | N-Acetylglucosamine | + | 47 | Gluconate | + |
| 23 | Amygdalin | + | 48 | 2-Ketogluconate | - |
| 24 | Arbutin | + | 49 | 5-Ketogluconate | - |
| + : Positive, - : Negative | | | | | |

[Experiment 5: Separation and identification of separated strain D]

Separated strain D was separated in the same manner as in Experiment 1 except that *Pandanus odoratissimus* was used as a separation source. Identification of separated strain D was performed in the same manner as in Experiment 2.

The nucleotide sequence of the 16S rRNA gene of separated strain D showed an identity of 99.87% to the nucleotide sequence of the 16S rRNA gene of *Lactobacillus pentosus* (JCM1558), an identity of 99.87% to the nucleotide sequence of the 16S rRNA gene of *Lactobacillus plantarum* subsp. *plantarum* (JCM1149), and an identity of 99.66% to the nucleotide sequence of the 16S rRNA gene of *Lactobacillus paraplantarum* (DSM10667). However, no microorganism had a 16S rRNA gene that completely matched the nucleotide sequence of the 16S rRNA gene of separated strain D.

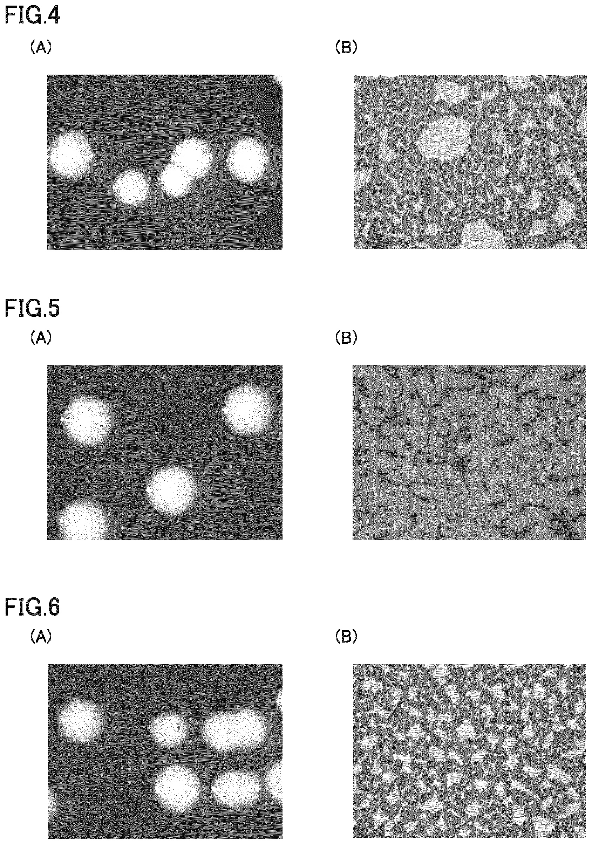

As shown in FIG. 4(A), separated strain D formed a circular colony. As shown in FIG. 4(B), separated strain D was positive for gram stainability. Table 7 and Table 8 show the results of a physiological and biochemical characterization test and a fermentability test for separated strain D. Separated strain D was a gram-positive rod without motility that did not form a spore, was negative for catalase reaction and oxidase reaction, and fermented glucose. These characteristics matched the characteristics of the genus *Lactobacillus,* to which the possibility of attribution was shown as a result of a 16S rDNA partial nucleotide sequence analysis. As a result of a fermentability test using an API kit, separated strain D fermented galactose, fructose, α-methyl-D-mannoside, melezitose, and the like and did not ferment glycerol, D-xylose, and the like. Further, separated strain D did not exhibit arginine dihydrolase activity and grew at 15°C. For *L. pentosus* and *L. plantarum* shown to be closely related as a result of a 16S rDNA partial nucleotide sequence analysis, these characteristics matched the characteristics of *L. plantarum* while being different from the characteristics of *L. pentosus* in that glycerol or D-xylose were not fermented. Accordingly, separated strain D turned out to be a new separated strain belonging to *Lactobacillus plantarum.* Separated strain D was internationally deposited as NITE BP-03200.

[Table 7]

| Test item | NITE BP-03200 |
|---|---|
| Culture temperature | 30°C |
| Cell morphology | Rod (0.7-0.8×2.0-3.0 μm) |
| Gram stainability | + |
| Presence or absence of spore | - |
| Motility | - |

(continued)

| Colony morphology | Medium | MRS agar medium |
|---|---|---|
| | Culture time | 48 hours |
| | Diameter | 1 to 3 mm |
| | Color Tones | Milky |
| | Shape | Circular |
| | Raised state | Lens shape |
| | Circumferential edge | Entire edge |
| | Surface shape, etc. | Smooth |
| | Transparency | Opaque |
| | Consistency | Butter-like |
| Growth temperature test | 15°C | + |
| | 37°C | + |
| | 45°C | - |
| Arginine dihydrolase activity | | - |
| Catalase reaction | | - |
| Oxidase reaction | | - |
| Acid/gas production from glucose (Acid production/gas production) | | + / - |
| O/F test (Oxidation/Fermentation) | | + / + |
| + : Positive, - : Negative | | |

[Table 8]

| Item | Substrate component | Test results | Item | Substrate component | Test results |
|---|---|---|---|---|---|
| 0 | Control | - | 25 | Esculin | + |
| 1 | Glycerol | - | 26 | Salicin | + |
| 2 | Erythritol | - | 27 | Cellobiose | + |
| 3 | D-Arabinose | - | 28 | Maltose | + |
| 4 | L-Arabinose | - | 29 | Lactose | + |
| 5 | Ribose | + | 30 | Melibiose | + |
| 6 | D-Xylose | - | 31 | Sucrose | + |
| 7 | L-Xylose | - | 32 | Trehalose | + |
| 8 | Adonitol | - | 33 | Inulin | - |
| 9 | β-Methyl-D-xylose | - | 34 | Melezitose | + |
| 10 | Galactose | + | 35 | Raffinose | + |
| 11 | Glucose | + | 36 | Starch | - |
| 12 | Fructose | + | 37 | Glycogen | - |
| 13 | Mannose | + | 38 | Xylitol | - |
| 14 | Sorbose | - | 39 | Gentiobiose | + |
| 15 | Rhamnose | - | 40 | D-Turanose | + |

(continued)

| Item | Substrate component | Test results | Item | Substrate component | Test results |
|---|---|---|---|---|---|
| 16 | Dulcitol | - | 41 | D-Lyxose | - |
| 17 | Inositol | - | 42 | D-Tagatose | - |
| 18 | Mannitol | + | 43 | D-Fucose | - |
| 19 | Sorbitol | + | 44 | L-Fucose | - |
| 20 | α-Methyl-D-mannoside | + | 45 | D-Arabitol | - |
| 21 | α-Methyl-D-glucoside | - | 46 | L-Arabitol | - |
| 22 | N-Acetylglucosamine | + | 47 | Gluconate | + |
| 23 | Amygdalin | + | 48 | 2-Ketogluconate | - |
| 24 | Arbutin | + | 49 | 5-Ketogluconate | - |
| + : Positive, - : Negative | | | | | |

[Experiment 6: Separation and identification of separated strain E]

Separated strain E was separated in the same manner as in Experiment 1 except that *Ficus variegata* was used as a separation source. Identification of separated strain E was performed in the same manner as in Experiment 2.
The nucleotide sequence of the 16S rRNA gene of separated strain E showed an identity of 99.93% to the nucleotide sequence of the 16S rRNA gene of *Lactobacillus pentosus* (JCM1558), an identity of 99.93% to the nucleotide sequence of the 16S rRNA gene of *Lactobacillus plantarum* subsp. *plantarum* (JCM1149), and an identity of 99.73% to the nucleotide sequence of the 16S rRNA gene of *Lactobacillus paraplantarum* (DSM10667). However, no microorganism had a 16S rRNA gene that completely matched the nucleotide sequence of the 16S rRNA gene of separated strain E.
As shown in FIG. 5(A), separated strain E formed a circular colony. As shown in FIG. 5(B), separated strain E was positive for gram stainability. Table 9 and Table 10 show the results of a physiological and biochemical characterization test and a fermentability test for separated strain E. Separated strain E was a gram-positive rod without motility that did not form a spore, was negative for catalase reaction and oxidase reaction, and fermented glucose. These characteristics matched the characteristics of the genus *Lactobacillus,* to which the possibility of attribution was shown as a result of a 16S rDNA partial nucleotide sequence analysis. As a result of a fermentability test using an API kit, separated strain E fermented galactose, fructose, melezitose, and the like and did not ferment glycerol, D-xylose, and the like. Further, separated strain E did not exhibit arginine dihydrolase activity and grew at 15°C. For *L. pentosus* and *L. plantarum,* to which attribution was suggested as a result of a 16S rDNA partial nucleotide sequence analysis, these characteristics matched the characteristics of *L. plantarum* while being different from the characteristics of *L. pentosus* in that glycerol or D-xylose were not fermented. Accordingly, separated strain E turned out to be a new separated strain belonging to *Lactobacillus plantarum.* Separated strain E was internationally deposited as NITE BP-03201.

[Table 9]

| Test item | NITE BP-03201 |
|---|---|
| Culture temperature | 30°C |
| Cell morphology | Rod (0.9-1.0×2.0-4.0 μm) |
| Gram stainability | + |
| Presence or absence of spore | - |
| Motility | - |

(continued)

| Colony morphology | Medium | MRS agar medium |
|---|---|---|
| | Culture time | 48 hours |
| | Diameter | 1 to 3 mm |
| | Color Tones | Milky |
| | Shape | Circular |
| | Raised state | Lens shape |
| | Circumferential edge | Entire edge |
| | Surface shape, etc. | Smooth |
| | Transparency | Opaque |
| | Consistency | Butter-like |
| Growth temperature test | 15°C | + |
| | 37°C | + |
| | 45°C | - |
| Arginine dihydrolase activity | | - |
| Catalase reaction | | - |
| Oxidase reaction | | - |
| Acid/gas production from glucose (Acid production/gas production) | | +/- |
| O/F test (Oxidation/Fermentation) | | +/+ |
| + : Positive, - : Negative | | |

[Table 10]

| Item | Substrate component | Test results | Item | Substrate component | Test results |
|---|---|---|---|---|---|
| 0 | Control | - | 25 | Esculin | + |
| 1 | Glycerol | - | 26 | Salicin | + |
| 2 | Erythritol | - | 27 | Cellobiose | + |
| 3 | D-Arabinose | - | 28 | Maltose | + |
| 4 | L-Arabinose | + | 29 | Lactose | + |
| 5 | Ribose | + | 30 | Melibiose | + |
| 6 | D-Xylose | - | 31 | Sucrose | + |
| 7 | L-Xylose | - | 32 | Trehalose | + |
| 8 | Adonitol | - | 33 | Inulin | - |
| 9 | β-Methyl-D-xylose | - | 34 | Melezitose | + |
| 10 | Galactose | + | 35 | Raffinose | + |
| 11 | Glucose | + | 36 | Starch | - |
| 12 | Fructose | + | 37 | Glycogen | - |
| 13 | Mannose | + | 38 | Xylitol | - |
| 14 | Sorbose | - | 39 | Gentiobiose | + |
| 15 | Rhamnose | - | 40 | D-Turanose | + |

(continued)

| Item | Substrate component | Test results | Item | Substrate component | Test results |
|------|---------------------|--------------|------|---------------------|--------------|
| 16 | Dulcitol | - | 41 | D-Lyxose | - |
| 17 | Inositol | - | 42 | D-Tagatose | - |
| 18 | Mannitol | + | 43 | D-Fucose | - |
| 19 | Sorbitol | + | 44 | L-Fucose | - |
| 20 | α-Methyl-D-mannoside | - | 45 | D-Arabitol | - |
| 21 | α-Methyl-D-glucoside | - | 46 | L-Arabitol | - |
| 22 | N-Acetylglucosamine | + | 47 | Gluconate | + |
| 23 | Amygdalin | + | 48 | 2-Ketogluconate | - |
| 24 | Arbutin | + | 49 | 5-Ketogluconate | - |
| + : Positive, - : Negative | | | | | |

[Experiment 7: Separation and identification of separated strain F]

Separated strain F was separated in the same manner as in Experiment 1 except that pinecone was used as a separation source. Identification of separated strain F was performed in the same manner as in Experiment 2.

The nucleotide sequence of the 16S rRNA gene of separated strain F showed an identity of 99.93% to the nucleotide sequence of the 16S rRNA gene of *Lactobacillus pentosus* (JCM1558), an identity of 99.93% to the nucleotide sequence of the 16S rRNA gene of *Lactobacillus plantarum* subsp. *plantarum* (JCM1149), and an identity of 99.73% to the nucleotide sequence of the 16S rRNA gene of *Lactobacillus paraplantarum* (DSM10667). However, no microorganism had a 16S rRNA gene that completely matched the nucleotide sequence of the 16S rRNA gene of separated strain F.

As shown in FIG. 6(A), separated strain F formed a circular colony. As shown in FIG. 6(B), separated strain F was positive for gram stainability. Table 11 and Table 12 show the results of a physiological and biochemical characterization test and a fermentability test for separated strain F. Separated strain F was a gram-positive rod without motility that did not form a spore, was negative for catalase reaction and oxidase reaction, and fermented glucose. These characteristics matched the characteristics of the genus *Lactobacillus,* to which the possibility of attribution was shown as a result of a 16S rDNA partial nucleotide sequence analysis. As a result of a fermentability test using an API kit, separated strain F fermented galactose, fructose, α-methyl-D-mannoside, melezitose, and the like and did not ferment glycerol, D-xylose, and the like. Further, separated strain F did not exhibit arginine dihydrolase activity and grew at 15°C. For *L. pentosus* and *L. plantarum,* to which attribution was suggested as a result of a 16S rDNA partial nucleotide sequence analysis, these characteristics matched the characteristics of *L. plantarum* while being different from the characteristics of *L. pentosus* in that glycerol or D-xylose were not fermented. Accordingly, separated strain F turned out to be a new separated strain belonging to *Lactobacillus plantarum.* Separated strain F was internationally deposited as NITE BP-03202.

[Table 11]

| Test item | NITE BP-03202 |
|-----------|---------------|
| Culture temperature | 30°C |
| Cell morphology | Rod (0.8-0.9×1.0-2.0 μm) |
| Gram stainability | + |
| Presence or absence of spore | - |
| Motility | - |

(continued)

| Test item | | NITE BP-03202 |
|---|---|---|
| Colony morphology | Medium | MRS agar medium |
| | Culture time | 48 hours |
| | Diameter | 1 to 2 mm |
| | Color Tones | Milky |
| | Shape | Circular |
| | Raised state | Lens shape |
| | Circumferential edge | Entire edge |
| | Surface shape, etc. | Smooth |
| | Transparency | Opaque |
| | Consistency | Butter-like |
| Growth temperature test | 15°C | + |
| | 37°C | + |
| | 45°C | - |
| Arginine dihydrolase activity | | - |
| Catalase reaction | | - |
| Oxidase reaction | | - |
| Acid/gas production from glucose (Acid production/gas production) | | +/- |
| O/F test (Oxidation/Fermentation) | | +/+ |
| + : Positive, - : Negative | | |

[Table 12]

| Item | Substrate component | Test results | Item | Substrate component | Test results |
|---|---|---|---|---|---|
| 0 | Control | - | 25 | Esculin | + |
| 1 | Glycerol | - | 26 | Salicin | + |
| 2 | Erythritol | - | 27 | Cellobiose | + |
| 3 | D-Arabinose | - | 28 | Maltose | + |
| 4 | L-Arabinose | + | 29 | Lactose | + |
| 5 | Ribose | + | 30 | Melibiose | + |
| 6 | D-Xylose | - | 31 | Sucrose | + |
| 7 | L-Xylose | - | 32 | Trehalose | + |
| 8 | Adonitol | - | 33 | Inulin | - |
| 9 | β-Methyl-D-xylose | - | 34 | Melezitose | + |
| 10 | Galactose | + | 35 | Raffinose | + |
| 11 | Glucose | + | 36 | Starch | - |
| 12 | Fructose | + | 37 | Glycogen | - |
| 13 | Mannose | + | 38 | Xylitol | - |
| 14 | Sorbose | - | 39 | Gentiobiose | + |

(continued)

| Item | Substrate component | Test results | Item | Substrate component | Test results |
|------|--------------------|--------------|------|--------------------|--------------|
| 15 | Rhamnose | - | 40 | D-Turanose | + |
| 16 | Dulcitol | - | 41 | D-Lyxose | - |
| 17 | Inositol | - | 42 | D-Tagatose | - |
| 18 | Mannitol | + | 43 | D-Fucose | - |
| 19 | Sorbitol | + | 44 | L-Fucose | - |
| 20 | α-Methyl-D-mannoside | + | 45 | D-Arabitol | - |
| 21 | α-Methyl-D-glucoside | + | 46 | L-Arabitol | - |
| 22 | N-Acetylglucosamine | + | 47 | Gluconate | + |
| 23 | Amygdalin | + | 48 | 2-Ketogluconate | - |
| 24 | Arbutin | + | 49 | 5-Ketogluconate | - |
| + : Positive, - : Negative | | | | | |

[Experiment 8: Evaluation test for antibacterial activity of the cell culture product]

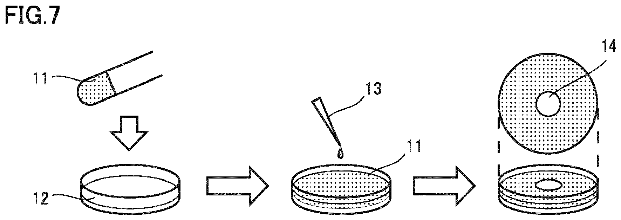

Whether or not the cell culture product of NITE BP-03197, NITE BP-03198, NITE BP-03199, NITE BP-03200, NITE BP-03201, or NITE BP-03202 had antibacterial activity was examined. The procedure of Experiment 8 will be described according to FIG. 7. *Ralstonia solanacearum* strains 1 to 6 (*Ralstonia solanacearum* and *Ralstoniapseudosolanacearum*) shown in Table 13 were used in the experiment. The turbidity of the preculture solution of each *Ralstonia solanacearum* strain was measured at a wavelength of 600 nm, followed by pour plating on a soft agar medium 11 to 0.1 OD units for *Ralstonia solanacearum* strain 1 and 0.4 OD units for other *Ralstonia solanacearum* strains. The amount of bacteria contained in 1 mL of a culture with OD600 = 1 was defined as 1 OD unit. Soft agar medium 11 containing each *Ralstonia solanacearum* strain was overlayed on an agar medium 12, and the surface was dried to prepare a growth medium of each *Ralstonia solanacearum* strain. NITE BP-03197, NITE BP-03198, NITE BP-03199, NITE BP-03200, NITE BP-03201, and NITE BP-03202 were each cultured in an MRS liquid medium at a temperature of 30°C, and bacterial cells were precipitated by centrifugation to prepare a cell culture supernatant (cell culture product) as a sample 13. 10 μL of sample 13 was dropped to the growth medium of each *Ralstonia solanacearum* strain, followed by culture at a temperature of 30°C for 20 hours under aerobic conditions. When the culture supernatant of bacteria has antibacterial activity against the *Ralstonia solanacearum* strain, a growth inhibition circle 14 is formed. As a control experiment, tetracycline (50 μg/mL) having antibacterial activity was used.

[Table 13]

|  | MAFF number | Scientific name | Separation source |
|--|-------------|-----------------|-------------------|
| *Ralstonia solanacearum* Strain 1 | 301823 | *Ralstonia pseudosolanacearum* | Green pepper, Sweet green pepper |
| *Ralstonia solanacearum* Strain 2 | 311121 | *Ralstonia pseudosolanacearum* | Strawberry |
| *Ralstonia solanacearum* Strain 3 | 730126 | *Ralstonia pseudosolanacearum* | Eggplant |
| *Ralstonia solanacearum* Strain 4 | 730133 | *Ralstonia pseudosolanacearum* | Tomato |
| *Ralstonia solanacearum* Strain 5 | 211534 | *Ralstonia solanacearum* | Tomato |
| *Ralstonia solanacearum* Strain 6 | - | *Ralstonia solanacearum* | Japanese Patent Laying-Open No. 5-39257 (SC-1801 Strain) |

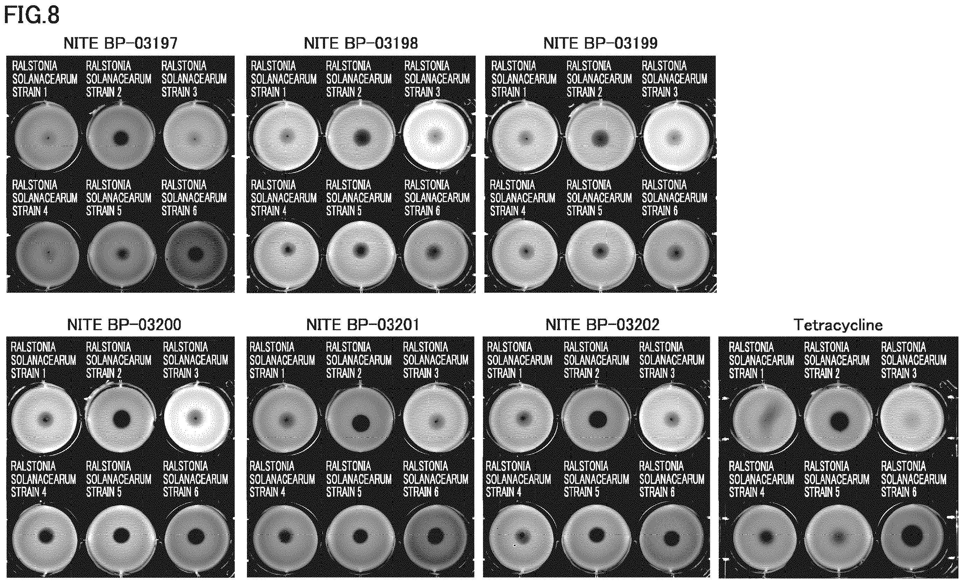

FIG. 8 shows the results of Experiment 8. By the culture supernatant of any of NITE BP-03197, NITE BP-03198, NITE BP-03199, NITE BP-03200, NITE BP-03201, or NITE BP-03202, a growth inhibition circle was formed in the growth medium of each of *Ralstonia solanacearum* strains 1 to 6. It was demonstrated that the cell culture product of any of NITE BP-03197, NITE BP-03198, NITE BP-03199, NITE BP-03200, NITE BP-03201, or NITE BP-03202 inhibit the growth of *Ralstonia solanacearum* and *Ralstoniapseudosolanacearum.*

[Experiment 9: Evaluation test for antibacterial activity of mixture of bacterial cells and the cell culture product]

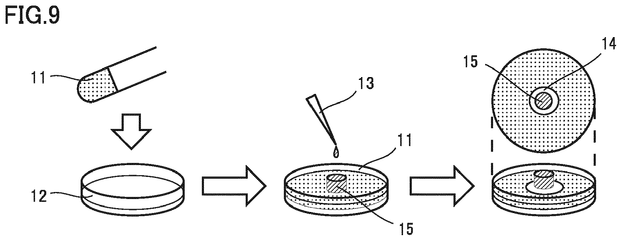

Whether or not the bacterial cells and the cell culture product of NITE BP-03197, NITE BP-03198, NITE BP-03199, NITE BP-03200, NITE BP-03201, or NITE BP-03202 had antibacterial activity was examined. Experiment 9 was the same as Experiment 8 except that a mixture of the bacterial cells and the culture supernatant was used as sample 13. FIG. 9 shows the experimental procedure. First, the growth medium of each *Ralstonia solanacearum* strain was prepared in the same manner as in Experiment 8. A mixture of the bacterial cells and the culture supernatant (cell culture product) obtained by culturing each of NITE BP-03197, NITE BP-03198, NITE BP-03199, NITE BP-03200, NITE BP-03201, and NITE BP-03202 in an MRS liquid medium at a temperature of 30°C was prepared as sample 13. A filter 15 immersed with 60 μL of sample 13 was left standing in the growth medium of each *Ralstonia solanacearum* strain, followed by culture at a temperature of 30°C for 20 hours under aerobic conditions. After culture, whether or not a growth inhibition circle was formed was checked.

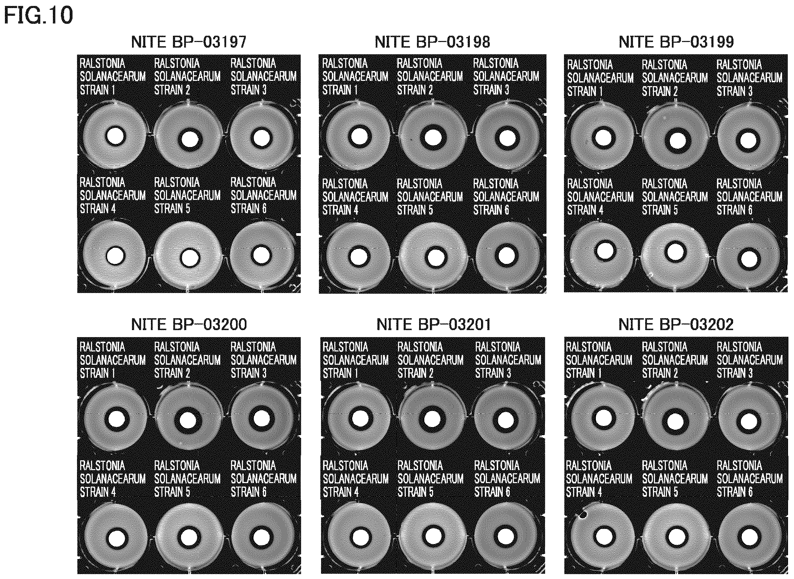

FIG. 10 shows the results of Experiment 9. By the mixture of the bacterial cells and the culture supernatant of any of NITE BP-03197, NITE BP-03198, NITE BP-03199, NITE BP-03200, NITE BP-03201, or NITE BP-03202, a growth inhibition circle was formed in the growth medium of each of *Ralstonia solanacearum* strains 1 to 6. It was demonstrated that the mixture of the bacterial cells and the cell culture product of any of NITE BP-03197, NITE BP-03198, NITE BP-03199, NITE BP-03200, NITE BP-03201, or NITE BP-03202 inhibit the growth of *Ralstonia solanacearum* and *Ralstoniapseudosolanacearum.*

[Experiment 10: Evaluation test for long-term antibacterial activity of mixture of bacterial cells and the cell culture product]

Whether or not the bacterial cells and the cell culture product of NITE BP-03197, NITE BP-03198, NITE BP-03199, NITE BP-03200, NITE BP-03201, or NITE BP-03202 had antibacterial activity over a long period of time was examined. Experiment 10 was performed in the same manner as in Experiment 9 except that a *Ralstonia solanacearum* strain 5 shown in Table 13 was used as a *Ralstonia solanacearum* strain, and that the culture period after filter 15 was left standing in the growth medium of the *Ralstonia solanacearum* strain was extended to 3 days, 7 days, or 15 days. As a control experiment, a lactic acid bacteria medium, an untreated medium, or tetracycline (50 μg/mL) was used.

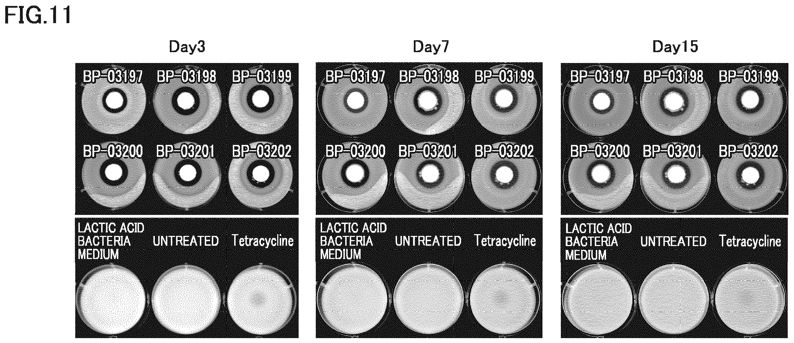

FIG. 11 shows the results of Experiment 10. Even 15 days after the filter immersed with the bacterial cells and the culture supernatant of NITE BP-03197, NITE BP-03198, NITE BP-03199, NITE BP-03200, NITE BP-03201, or NITE BP-03202 was left standing in the growth medium of the *Ralstonia solanacearum* strain, a growth inhibition circle was formed. It turned out that the mixture of the bacterial cells and the cell culture product of any of NITE BP-03197, NITE BP-03198, NITE BP-03199, NITE BP-03200, NITE BP-03201, or NITE BP-03202 inhibit the growth of the *Ralstonia solanacearum* strain over a long period of time.

[Experiment 11: Evaluation test for antibacterial activity against *Ralstonia solanacearum* in nutrient solution]

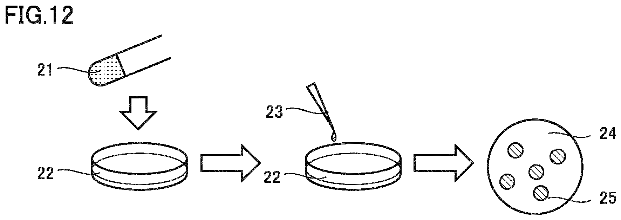

Whether or not the cell culture product of NITE BP-03197, NITE BP-03198, NITE BP-03199, NITE BP-03200, NITE BP-03201, or NITE BP-03202 inhibited the growth of the *Ralstonia solanacearum* strain in a nutrient solution was examined. The experimental procedure will be described according to FIG. 12. NITE BP-03197, NITE BP-03198, NITE BP-03199, NITE BP-03200, NITE BP-03201, and NITE BP-03202 were each cultured in an MRS liquid medium at a temperature of 30°C, and bacterial cells were precipitated by centrifugation to prepare a cell culture supernatant (cell culture product) as a sample 23. As a nutrient solution 22, an OAT A prescription, available from OAT Agrio Co., Ltd. (aqueous solution containing 1.5 g/L of OAT house 1 and 1.0 g/L of OAT house 2) was used. A *Ralstonia solanacearum* 21 was suspended in nutrient solution 22 to 0.01 OD units, to prepare nutrient solution 22 contaminated with the *Ralstonia solanacearum* strain. 10 μL of sample 23 was added dropwise to 90 μL of nutrient solution 22 and stirred, followed by culture at a temperature of 30°C for 5 hours. The culture solution was diluted 100-fold, applied onto an agar medium 24 for detecting the *Ralstonia solanacearum* strain, and further cultured a temperature of 30°C for 48 hours. The number of colonies 25 on agar medium 24 after culture was counted. As a *Ralstonia solanacearum* strain, *Ralstonia solanacearum* strain 5 shown in Table 13 was used. As a control experiment, a lactic acid bacteria medium or tetracycline (50 μg/mL) was used instead of a sample.

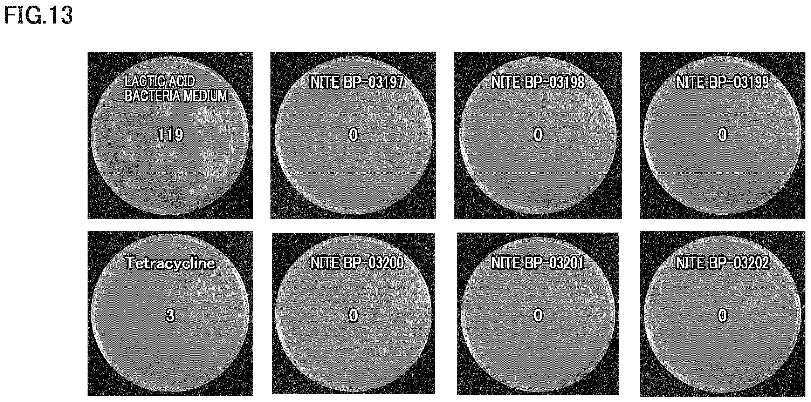

FIG. 13 shows the results of Experiment 11. In the nutrient solution to which the cell culture supernatant of NITE BP-03197, NITE BP-03198, NITE BP-03199, NITE BP-03200, NITE BP-03201, or NITE BP-03202 was added dropwise, the generation of colonies of the *Ralstonia solanacearum* strain was inhibited. It turned out that the *Ralstonia solanacearum* strain in the nutrient solution was killed or inhibited from growth by the cell culture product of NITE BP-03197, NITE BP-03198, NITE BP-03199, NITE BP-03200, NITE BP-03201, or NITE BP-03202.

[Experiment 12: Evaluation test for antibacterial activity against *Ralstonia solanacearum* in soil]

Whether or not the bacterial cells of NITE BP-03197, NITE BP-03198, NITE BP-03199, NITE BP-03200, NITE BP-03201, or NITE BP-03202 can inhibit the growth of the *Ralstonia solanacearum* strain in soil is examined. To 50 g of soil (culture soil for flowers and vegetables, available from Akagi Engei), is added 2.5 mL of the bacterial solution of *Ralstonia solanacearum* strain 5 adjusted with distilled water (available from Otsuka Pharmaceutical Factory, Inc.) to 0.06 OD units, to prepare contaminated soil. 1 g of a reduction material (organic matter of molasses, or the like, available from Sunpillars Co., Ltd., Omalass 95) and the bacterial cells of NITE BP-03197, NITE BP-03198, NITE BP-03199, NITE BP-03200, NITE BP-03201, or NITE BP-03202 are mixed with soil, followed by culture at a temperature of 30°C for 14 days under anaerobic conditions. 2.5 mL of the bacterial cells obtained by culturing each bacteria in an MRS liquid medium at a temperature of 30°C and adjusted with distilled water (available from Otsuka Pharmaceutical Factory, Inc.) to 0.5 OD units are mixed per 50 g of soil. The reduction material used is one used as a soil fumigant. A DNA of a microorganism contained in the soil is purified from the soil using an ISOII, for Beads Beating Kit (available from NIPPON GENE CO., LTD.) and amplified by PCR using a common primer set for *Ralstonia solanacearum* (759: GTCGCCGTCAACTCAACTTTCC (SEQ ID No: 2) and 760: GTCGCCGTCAGCAATGCGGAATCG (SEQ ID No: 3)) and a PCR primer set specific to *Ralstonia solanacearum* strain 5 (Nmult21: 1F CGTTGATGAGGCGCGCAATTT (SEQ ID No: 4) and Nmult21: RR TTCGCTTGACCCTATAACGAGT (SEQ ID No: 5)). PCR amplification is performed using KOD-Plus-Ver.2 DNA polymerase (available from TOYOBO CO., LTD.), and the amplified product after PCR is purified. The PCR amplified product purified is electrophoresed on 4% E-Gel (available from Thermo Fisher Scientific K.K.), and the band of 144 base pairs amplified with the PCR primer set specific to *Ralstonia solanacearum* strain 5 is observed with an image analyzer LAS-3000 (available from FUJIFILM Corporation).
The band of 144 base pairs specific to *Ralstonia solanacearum* strain 5 decreases in the soil mixed with the bacterial cells of NITE BP-03197, NITE BP-03198, NITE BP-03199, NITE BP-03200, NITE BP-03201, or NITE BP-03202. The *Ralstonia solanacearum* strain in the soil is killed or inhibited from growth by the bacterial cells of NITE BP-03197, NITE BP-03198, NITE BP-03199, NITE BP-03200, NITE BP-03201, or NITE BP-03202.

[Experiment 13: Test for effect of controlling bacterial wilt in nutriculture]

Whether or not bacterial wilt was controlled in nutriculture by adding the cell culture productof NITE BP-03199, NITE BP-03200, or NITE BP-03201 to a nutrient solution was examined. First, a tomato (variety: REIYOH) was cultivated to the 4th to 5th leaf stage using LED Planter Greenteria (available from K.K.DeAGOSTINI JAPAN) in a nutrient solution of an OAT A prescription under conditions at a temperature of 25°C and a humidity of 20 to 30% with a photoperiod: 16 hours bright/8 hours dark. Eight plants of tomato were used for each of the test area and the control area.
In the test area, the cell culture supernatant prepared from NITE BP-03201 culture with OD600 = 10 was added to the nutrient solution in an amount of 1/100 of the nutrient solution before inoculation of *Ralstonia solanacearum.* In the control area, an MRS medium was added to the nutrient solution in an amount of 1/100 of the nutrient solution before inoculation of *Ralstonia solanacearum.* Then, the bacterial solution of *Ralstonia solanacearum* strain 5 was added to the nutrient solution to $2 \times 10^7$ cfu/mL. Thereafter, *Ralstonia solanacearum* was inoculated by cutting the tip of the root of the tomato. Over the course of 10 to 12 days starting from the day of inoculation until the completion of the test, the symptom expression appearing on the plant was evaluated into the following 5-point scale according to the evaluation criteria of Non Patent Literature 1.

    0: No disease
    1: One leaf wilted
    2: Two leaves or more wilted
    3: Leaves except parietal lobe wilted
    4: Whole body wilted or withered

$$\text{Disease index} = (0 \times N0 + 1 \times N1 + 2 \times N2 + 3 \times N3 + 4 \times N4)/4 \times (N0 + N1 + N2 + N3 + N4)$$

N0 to N4 each represent the number of plants that was given such a numerical point. The disease index represents the degree of damage caused by the disease. A larger value of the disease index means that the disease progresses more.

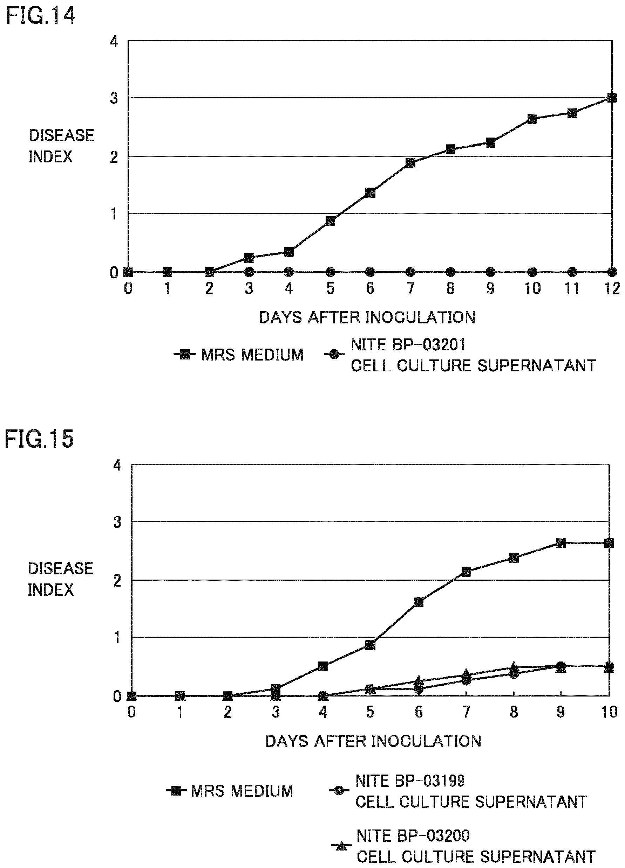

FIG. 14 shows a change over time in disease index after inoculation of the *Ralstonia solanacearum.* A severe symptom expression was seen in the control area, whereas such a symptom expression was not seen in the test area. Accordingly, it turned out that the cell culture product of NITE BP-03201 has an effect of controlling bacterial wilt in nutriculture.

FIG. 15 shows the results of examining whether or not bacterial wilt was controlled by adding the cell culture supernatant of NITE BP-03199 or NITE BP-03200 to the nutrient solution in an amount of 1/100 of the amount of nutrient solution in the same manner as above. A severe symptom expression was seen in the control area, whereas the disease index was clearly low in the test area. Accordingly, it turned out that the cell culture product of NITE BP-03199 or NITE BP-03200 also exhibits the effect of controlling bacterial wilt in nutriculture.

[Experiment 14: Test for effect of controlling bacterial wilt in soil culture]

Whether or not bacterial wilt can be controlled by applying the cell culture products of NITE BP-03200 in soil culture is examined. One week before planting, the cell culture supernatant of NITE BP-03200 is diluted 10-fold, 50-fold, or 100-fold, and 50 mL of each is irrigated per young plant. Then, the *Ralstonia solanacearum* strain is inoculated by planting the tomato seedling in *Ralstonia solanacearum*-contaminated field. After planting in the field, the cell culture supernatant of NITE BP-03200 is diluted 10-fold, 50-fold, or 100-fold, and 500 mL of each is irrigated per young plant at weekly intervals. The symptom expression appearing on the plant is compared between the agent-treated area and the untreated area, to evaluate the control effect.

Withering or wilting is seen in the untreated area, whereas such a symptom expression is inhibited in the area irrigated with the cell culture supernatant of NITE BP-03200. Therefore, it is found that the cell culture products of NITE BP-03200 exhibits the effect of controlling bacterial wilt in soil cultivation.

REFERENCE SIGNS LIST

11: Soft agar medium, 12: Agar medium, 13: Sample, 14: Growth inhibition circle, 15: Filter, 21: *Ralstonia solanacearum,* 22: Nutrient solution, 23: Sample, 24: Agar medium, 25: Colony

**Claims**

1. A bacterium having a 16S rRNA gene that contains a nucleotide sequence with 98.80 % or more identity to the nucleotide sequence of SEQ ID No: 1 and having the ability to assimilate mannitol and the ability to control a plant disease.

2. A bacterium having a 16S rRNA gene that contains a nucleotide sequence with 99.90 % or more identity to the nucleotide sequence of SEQ ID No: 1 and having the ability to control a plant disease.

3. The bacterium according to claim 1 or 2, having a 16S rRNA gene that contains the nucleotide sequence of SEQ ID No: 1.

4. A bacterium with Accession Number of NITE BP-03197, NITE BP-03198, NITE BP-03199, NITE BP-03200, NITE BP-03201, or NITE BP-03202.

5. Bacterial cells or a cell culture product of the bacterium according to any one of claims 1 to 4, or an extract thereof.

6. A control agent against a plant disease caused by *Ralstonia solanacearum* or *Ralstoniapseudosolanacearum,* comprising bacterial cells or a cell culture product of the bacterium according to any one of claims 1 to 4, or an extract thereof.

7. A method for controlling a plant disease caused by *Ralstonia solanacearum* or *Ralstoniapseudosolanacearum,* comprising applying the control agent according to claim 6.

8. A disinfectant for a plant, a nutrient solution, soil, a nutriculture material, or a soil culture material contaminated by *Ralstonia solanacearum* or *Ralstonia pseudosolanacearum,* comprising bacterial cells or a cell culture product of the bacterium according to any one of claims 1 to 4, or an extract thereof.

9. A method for disinfecting a plant, a nutrient solution, soil, a nutriculture material, or a soil culture material contaminated by *Ralstonia solanacearum* or *Ralstonia pseudosolanacearum,* comprising using the disinfectant according to claim 8.

10. A growth inhibitor for *Ralstonia solanacearum* or *Ralstonia pseudosolanacearum,* comprising bacterial cells or a cell culture product of the bacterium according to any one of claims 1 to 4, or an extract thereof.

11. A method for inhibiting growth of *Ralstonia solanacearum* or *Ralstonia pseudosolanacearum,* comprising using the growth inhibitor according to claim 10.

12. A method for cultivating a plant, comprising cultivating a plant in a nutrient solution or soil that contains bacterial cells or a cell culture product of the bacterium according to any one of claims 1 to 4, or an extract thereof.

13. A control agent against a plant disease caused by *Ralstonia solanacearum* or *Ralstoniapseudosolanacearum,* comprising bacterial cells or a cell culture product of bacterium belonging to *Lactobacillus plantarum,* or an extract thereof.

14. A method for controlling a plant disease caused by *Ralstonia solanacearum* or *Ralstoniapseudosolanacearum,* comprising applying the control agent according to claim 13.

FIG.1

(A)

(B)

FIG.2

(A)

(B)

FIG.3

(A)

(B)

FIG.4

(A)

(B)

FIG.5

(A)

(B)

FIG.6

(A)

(B)

FIG.7

FIG.8

FIG.9

FIG.10

FIG.11

FIG.12

# FIG.13

FIG.14

DISEASE INDEX

DAYS AFTER INOCULATION

-■- MRS MEDIUM  -●- NITE BP-03201
CELL CULTURE SUPERNATANT

FIG.15

DISEASE INDEX

DAYS AFTER INOCULATION

-■- MRS MEDIUM  -●- NITE BP-03199
CELL CULTURE SUPERNATANT

-▲- NITE BP-03200
CELL CULTURE SUPERNATANT

**EP 4 174 167 A1**

<table>
<tr><td colspan="2" align="center"><strong>INTERNATIONAL SEARCH REPORT</strong></td><td>International application No.<br>PCT/JP2021/023736</td></tr>
</table>

**A. CLASSIFICATION OF SUBJECT MATTER**
Int.Cl. C12N1/20(2006.01)i, A01P3/00(2006.01)i, A01N63/20(2020.01)i
FI: C12N1/20AZNA, A01P3/00, A01N63/20

According to International Patent Classification (IPC) or to both national classification and IPC

**B. FIELDS SEARCHED**

Minimum documentation searched (classification system followed by classification symbols)
Int.Cl. C12N1/20, A01P3/00, A01N63/20

Documentation searched other than minimum documentation to the extent that such documents are included in the fields searched
Published examined utility model applications of Japan    1922-1996
Published unexamined utility model applications of Japan  1971-2021
Registered utility model specifications of Japan          1996-2021
Published registered utility model applications of Japan  1994-2021

Electronic data base consulted during the international search (name of data base and, where practicable, search terms used)
JSTPlus/JMEDPlus/JST7580(JDreamIII), CAplus/MEDLINE/EMBASE/BIOSIS(STN)

**C. DOCUMENTS CONSIDERED TO BE RELEVANT**

| Category* | Citation of document, with indication, where appropriate, of the relevant passages | Relevant to claim No. |
|---|---|---|
| Y<br>A | WO 2006/025167 A1 (KYOTO PREFECTURE) 09 March 2006 (2006-03-09), claims, examples, paragraphs [0019], [0023], etc. | 13, 14<br>1-12 |
| Y | WO 2019/068887 A1 (AGROLAC, S.A.) 11 April 2019 (2019-04-11), table 1 | 13, 14 |
| A | IKRAM, M. et al., Novel antimicrobial and antioxidative activity by endophytic penicillium roqueforti and trichoderma reesei isolated from solanum surattense, Acta Physiologiae Plantarum, 2019, 41:164, abstract | 1-14 |
| A | JP 2009-201459 A (KYOTO PREFECTURE) 10 September 2009 (2009-09-10), claims, examples | 1-14 |

☐ Further documents are listed in the continuation of Box C.          ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>03 September 2021 | Date of mailing of the international search report<br>14 September 2021 |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

INTERNATIONAL SEARCH REPORT
Information on patent family members

International application No.

PCT/JP2021/023736

| | | | |
|---|---|---|---|
| WO 2006/025167 A1 | 09 March 2006 | US 2009/0169530 A1 claims, examples | |
| WO 2019/068887 A1 | 11 April 2019 | EP 3467097 A1 table 1 | |
| JP 2009-201459 A | 10 September 2009 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009201459 A
- JP 2012211124 A
- JP 5039257 A

**Non-patent literature cited in the description**

- *J Gen Plant Pathol,* 2004, vol. 70, 115-119
- Gene analysis method, 16S rRNA gene sequencing. **NAKAGAWA YASUYOSHI et al.** Classification and identification of actinomycetes. the Center for Academic Societies Japan, 2001, vol. 88, 117
- **BARROW ; FELTHAM.** Cowan and Steel's Manual for the Identification of Medical Bacteria. Cambridge University Press, 1993
- Lactobacillus. **HAMMES WP ; HERTEL C et al.** Bergey's Manual of Systematics of Archaea and Bacteria. John Wiley & Sons, 2015